(19) Europäisches Patentamt / European Patent Office / Office européen des brevets

(11) **EP 3 825 737 B1**

(12) **EUROPÄISCHE PATENTSCHRIFT**

(45) Veröffentlichungstag und Bekanntmachung des Hinweises auf die Patenterteilung:
**19.02.2025   Patentblatt 2025/08**

(21) Anmeldenummer: **20209340.7**

(22) Anmeldetag: **23.11.2020**

(51) Internationale Patentklassifikation (IPC):
**G01T 1/29** (2006.01)

(52) Gemeinsame Patentklassifikation (CPC):
**G01T 1/2907**

(54) **VERFAHREN UND VORRICHTUNG ZUR MEHRDIMENSIONALEN RICHTUNGSMESSUNG VON GAMMASTRAHLUNG IM FERNFELD**

METHOD AND DEVICE FOR MULTIDIMENSIONAL DIRECTIONAL MEASUREMENT OF GAMMA RADIATION IN THE FAR FIELD

PROCÉDÉ ET DISPOSITIF DE MESURE DIRECTIONNELLE MULTIDIMENSIONNELLE DU RAYONNEMENT GAMMA DANS LE CHAMP LOINTAIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.11.2019   DE 102019131696
22.11.2019   DE 102019131695**

(43) Veröffentlichungstag der Anmeldung:
**26.05.2021   Patentblatt 2021/21**

(73) Patentinhaber: **Hellma Materials GmbH
07745 Jena (DE)**

(72) Erfinder: **PETRAK, Sibylle
07743 Jena (DE)**

(74) Vertreter: **Fritzsche, Thomas
Fritzsche Patent
Naupliastraße 110
81545 München (DE)**

(56) Entgegenhaltungen:
**US-A1- 2009 256 080**

• **GUEORGUIEV A ET AL: "A novel method to determine the directionality of radiation sources with two detectors based on coincidence measurements", NUCLEAR SCIENCE SYMPOSIUM CONFERENCE RECORD (NSS/MIC), 2010 IEEE, IEEE, 30 October 2010 (2010-10-30), pages 1525 - 1530, XP032054306, ISBN: 978-1-4244-9106-3, DOI: 10.1109/NSSMIC.2010.5874031**

## Beschreibung

Die Erfindung betrifft ein Verfahren sowie eine Vorrichtung zur Strahlungsdetektion, insbesondere ein richtungsmessendes Detektorsystem zur Messung der Richtungsverteilung der Strahlungsintensität. Das Verfahren unterstützt die 3-dimensionale wie auch die 2-dimensionale Richtungsmessung von einer und mehreren Strahlungsquellen im Fernfeld.

Im ABC- und Strahlenschutz gibt es eine Nachfrage nach handlichen, kompakten Strahlungsmessgeräten, die Richtungsinformationen bereitstellen können. Anders als bei der Bildgebung in der Medizin, wo Aktivitätskonzentrationen dargestellt werden, bedeutet Richtungsmessung hier, die Richtungen von einer oder mehreren Strahlungsquellen zu ermitteln, um damit das Auffinden der Strahlungsquellen zu unterstützen.

Kompakte Gammaspektrometer und Nuklid-Identifiziergeräte gehören zum Stand der Technik. Solche Geräte können jedoch den Einfallswinkel der Strahlung nicht bestimmen.

Eine Ortung von Strahlungsquellen im Gelände ist derzeit nur möglich, wenn Einsatzkräfte mit einem Strahlungsmessgerät das gesamte Gebiet ablaufen oder abfahren, um so eine Intensitätsverteilung zu erstellen. Das Verfahren ist sehr aufwendig und auch nicht immer anwendbar, wenn z.B. Strahlungsquellen in unzugänglichem Gelände geortet werden sollen.

Aus dem klinischen Umfeld sind komplexe, aus vielen Einzeldetektoren zusammengesetzte Geräte bekannt, die zur medizinischen Bildgebung eingesetzt werden. Diese Geräte sind komplex und benötigen komplexe Algorithmen für die Datenauswertung, die beträchtliche Rechenkapazitäten erfordern. Die Algorithmen berechnen die Aktivitätskonzentrationen eines Radiopharmakons in einem Patienten in unmittelbarer Nähe zu den Strahlungsdetektoren (Nahfeld). Grundsätzlich können diese Algorithmen auch für die genannten kompakten richtungsauflösenden Geräte verwendet werden, insofern sie an die Einsatzbedingungen im ABC- und Strahlenschutz angepasst werden.

Dazu müssen die Verfahren vom Nahfeld auf das Fernfeld umgestellt werden. Der Rechenaufwand muss soweit reduziert werden, dass eine Datenverarbeitung in Echtzeit parallel zur Datenakquisition möglich ist, so dass die Ergebnisse live während der Messung verfügbar sind. Ferner bestehen Unterschiede bezüglich der Strahlungsintensität. Beim Aufspüren radioaktiver Strahlungsquellen liegen die am Detektionsort registrierten Strahlungsintensitäten häufig weit unter den in der Medizin üblichen. Dies stellt erhöhte Anforderungen an die Algorithmen, die trotz statistischer Beschränkungen der Datensätze zuverlässige und reproduzierbare Ergebnisse liefern sollen. Die Richtungsmessung soll auch dann noch verlässlich funktionieren, wenn die Strahlungsintensität der zu vermessenen Quellen unter dem Niveau des natürlichen Strahlungshintergrundes liegt. Es ist daher erforderlich, dass der natürliche Strahlungshintergrund in der Auswertung berücksichtigt wird. Für das Lokalisieren von Strahlungsquellen im Gelände werden also schnelle Verfahren benötigt, die ein Strahlungsfernfeld rekonstruieren, auch bei niedriger Zählerstatistik einsetzbar sind und den Einfluss von natürlicher Strahlung berücksichtigen.

Es sind mehrere Verfahren bekannt, die für die Lokalisation von Strahlungsquellen im Gelände entwickelt wurden. Die US2012/0043467 beschreibt ein Verfahren für Single Plane Compton Kameras, das zur Richtungsmessung einer Strahlungsquelle geeignet ist. Dieses Verfahren hat jedoch den Nachteil, dass es auf die Richtungsmessung einer Strahlungsquelle pro Radionuklid beschränkt ist und keine Richtungsverteilung von mehreren Strahlungsquellen messen kann. Ferner beschreibt die US 8,461,547 B2 ein Verfahren, das die Richtungsinformationen von Compton Streuprozessen nutzt, um die Richtung einer Strahlungsquelle zu bestimmen. Aus GUEORGUIEV A ET AL: "A novel method to determine the directionality of radiation sources with two detectors based on coincidence measurements",NUCLEAR SCIENCE SYMPOSIUM CONFERENCE RECORD (NSS/MIC), 2010 IEEE, IEEE, 30. Oktober 2010, Seiten 1525-1530, DOI: 10.1109/NSSMIC.2010.5874031ISBN: 978-1-4244-9106-3, ist ein Verfahren zur Bestimmung der Richtung von Strahlungsquellen mithilfe von zwei Detektoren und basierend auf Koinzidenzmessungen bekannt. Es wird ein Set von Koinzidenzereignissen jedes Detektors in ein verschiedene Spektren aufgenommen. Die Energieverteilung wird statistisch analysiert. Der Mittelwert jeder Verteilung ist proportional zu $\theta$ aus der Compton Streuformel $f(\theta)=(E_L-E_R)/(E_L+E_R)$.

Die Verfahren nach dem Stand der Technik machen dabei keinen Gebrauch von Bildrekonstruktionsverfahren; vielmehr kommen einfache statistische Auswertetechniken zum Einsatz.

Die Aufgabe hat daher zum Ziel ein Verfahren für kompakte, richtungsauflösende Strahlungsmessgeräte bereit zu stellen, das auch für Verbundsysteme aus mehreren Strahlungsdetektoren und für segmentierte Strahlungsdetektoren einsetzbar ist.

Die Erfindung hat daher auch zum Ziel, die Nachteile der Verfahren aus dem Stand der Technik zu überwinden. Insbesondere soll eine verlässliche und effiziente Ortung von mehreren Strahlungsquellen ohne Beschränkung auf die Anzahl der Strahlungsquellen bereitgestellt werden.

Die Aufgaben werden durch das Verfahren und die Vorrichtung gemäß dem unabhängigen Ansprüchen gelöst. In den Unteransprüchen sind vorteilhafte Weiterbildungen und bevorzugte Ausführungen angegeben.

Das erfindungsgemäße Verfahren zur mehrdimensionalen Richtungsmessung von Gammastrahlung im Fernfeld mittels einer Gruppe von mehreren energiediskriminierenden, miteinander synchronisierten Detektoren zur Erfassung von Strahlung zeichnet sich dadurch aus, dass das Verfahren uni- und bidirektionale Compton Streuprozesse nutzt und Lookup-Tabellen $LUT^{SK}$, einen definierten Funktionswert $f(E1,E2)$, eine Liste von definierten Detektorpaaren mit einer Identifikationsnummer *i* für definierte Detektorpaare und eine oder mehrere Häufigkeitsverteilungen *Y* zur Erfassung von Messwerten verwendet. Das erfindungsgemäße Verfahren umfasst die folgenden Schritte:

**a) Einrichten** des Detektorsystems für eine Messung, folgende Schritte umfassend:

- **Erstellen** einer Liste mit definierten Detektorpaaren, wobei die definierten Detektorpaare alle Paare umfassen, welche kombinatorisch aus der Menge der Detektoren gebildet werden und jedes Paar mindestens einen Detektor aus einem Material mit einer Ordnungszahl von $Z_{eff} > 30$ enthält, und diese mit einer ID-Nummer *i* versieht;

- **Verschalten** aller Detektoren in einer Koinzidenzschaltung derart, dass Koinzidenzereignisse in allen definierten Detektorpaaren *i* = 1, ..., *I* erfasst werden;

- **Kennzeichnen** der beiden Detektoren eines jeden definierten Detektorpaares *i* mit den Nummern 1 bzw. 2, wobei der Detektor mit der niedrigeren Ordnungszahl die Nummer 1 und derjenige mit der höheren Ordnungszahl die Nummer 2 erhält, wenn beide Detektoren aus dem gleichen Material bestehen, wird die Kennzeichnung als 1 bzw. 2 willkürlich getroffen;

- **Definieren** einer Funktion f(E1,E2) = (E2-E1) / (E1+E2) welche aus zwei Energiewerten E1 und E2 berechnet wird;

**b) Akquirieren von Messwerten** von Koinzidenzereignissen, wenn in je zwei Detektoren von allen definierten Detektorpaaren *i* gleichzeitig Wechselwirkungen stattfinden, wobei die Messwerte einer Strahlungsverteilung im Fernfeld entstammen und die Messwerte die in den Detektoren gemessenen Wechselwirkungsenergien E1 und E2 der Strahlung sind;

**c) Assoziieren** von Koinzidenzereignissen mit einer Identifikationsnummer *i*;

**d) Berechnen** des Funktionswertes f(E1,E2) aus zwei Energiewerten E1, E2 pro Koinzidenzereignis mit der in Schritt a) definierten Funktion f(E1,E2);

**e) Erfassen** der Koinzidenzereignisse entsprechend ihrer Identifikationsnummer *i* und ihrer Funktionswerte f(E1,E2) in einer oder mehreren Häufigkeitsverteilungen *Y*, wobei für jedes Radionuklid eine separate Häufigkeitsverteilung *Y* vorliegt,

**f) Berechnen** einer oder mehrerer Richtungsverteilungen *X* aus den Häufigkeitsverteilungen *Y* mit einem statistischen Bildrekonstruktionsverfahren der Emissionstomographie unter Nutzung von Lookup-Tabellen $LUT^{SK}$, wobei für jedes Radionuklid eine separate Richtungsverteilung *X* vorliegt.

Das erfindungsgemäße Verfahren zur Richtungsmessung einer Strahlungsverteilung im Fernfeld gilt gleichermaßen für Verbundsysteme aus mehreren Strahlungsdetektoren als auch für segmentierte Strahlungsdetektoren. In einem Fall wird eine Gruppe aus mehreren Strahlungsdetektoren miteinander verschaltet, im anderen Fall ist das aktive Detektormedium in mehrere Einheiten unterteilt. Die Gruppe der Strahlungsdetektoren bzw. der segmentierte Einzeldetektor bilden jeweils ein zeitlich synchronisiertes System. Jeder Detektor bzw. jedes Segment kann die Energie der Strahlung messen, welche durch Wechselwirkung dort deponiert wird und kann mit anderen Detektoren bzw. Segmenten synchronisiert werden. Im Sinne der Erfindung wird unter einem Detektor ein Strahlungsdetektor bzw. ein Detektorsegment verstanden, welches Strahlung detektieren und individuell ausgelesen werden kann.

Es wird davon ausgegangen, dass das Messsystem mit Datenerfassung im List-Mode ausgestattet ist und die Daten eines jeden Detektors bzw. jeden Segments verarbeiten kann.

Das erfindungsgemäße Verfahren ist insbesondere zur stationären bzw. quasistationären Richtungsmessung geeignet wobei hauptsächlich zwei Ausführungen nämlich die 3-dimensionale Richtungsmessung und die 2-dimensionale Richtungsmessung von besonderem Vorteil sind.

Die 2-dimensionale Richtungsmessung ist ein Spezialfall der Richtungsmessung, wenn alle Strahlungsquellen in einer Ebene liegen, beispielsweise in der horizontalen Ebene. Gruppen von planaren Strahlungsdetektoren stellen für die 2-dimensionale Richtungsmessung geeignete Gerätetypen dar. Das Gerät ist dabei so orientiert, dass die Detektionsebene mit der Ebene der Strahlungsquellen identisch ist.

Das erfindungsgemäße Verfahren zur Richtungsmessung wird auch richtungsaufgelöste Koinzidenz-Spektroskopie genannt. In der Koinzidenz-Spektroskopie werden Koinzidenzen untersucht, bei denen in jeweils zwei Detektoren (oder Segmenten) des Messgeräts gleichzeitig Wechselwirkungen stattfinden. Paare aus jeweils zwei Detektoren sind die abbildenden Grundelemente der Richtungsmessung. Die in einem koinzidenten Detektorpaar registrierten Energieeinträge E1 und E2 werden gemeinsam mit der räumlichen Orientierung des Detektorpaars betrachtet.

Die richtungsaufgelöste Koinzidenz-Spektroskopie macht Gebrauch von zwei Wechselwirkungsprozessen von Gammastrahlung: der Compton-Streuung und der photoelektrischen Absorption.

Das Messgerät wird als Kombination von Detektorpaaren aufgefasst. Es sollen zunächst einige Definitionen zu den Detektorpaaren getroffen werden.

Es werden diejenigen Detektorpaare ausgewählt, die für die Richtungsmessung genutzt werden sollen. Bei der Auswahl ist es hilfreich, zunächst die Materialien zu betrachten, aus denen die Detektoren aufgebaut sind. Es genügt, die Materialien in zwei Klassen einzuteilen. Eine Gruppe wird von Materialien mittlerer bis hoher Ordnungszahl gebildet. Im Sinne der Erfindung wird unter mittlerer bis hoher Ordnungszahl eine Zahl $Z_{eff}$ von größer als 30 verstanden. Liegen die Detektormaterialien als chemische Verbindungen vor, so wird unter $Z_{eff}$ die effektive Ordnungszahl verstanden, d.h. die mittlere Ordnungszahl aller in der Verbindung enthaltenen Elemente unter Berücksichtigung der Atommassen der Elemente und ihrer stöchiometrischen Zusammensetzung.

Zu den Detektormaterialien mittlerer bis hoher Ordnungszahl $Z_{eff}$ > 30 gehören beispielsweise NaI, ein häufig genutztes Material in Szintillationsdetektoren, und auch die Halogenide der seltenen Erden wie $CeBr_3$, $LaBr_3$, $LaCl_3$ und $La(Br_xCl_{1-x})_3$. Unter den Halbleitermaterialien mittlerer bis hoher Ordnungszahl sind insbesondere Ge, GaAs, CdTe und CdZnTe zu nennen. Diese Materialien zeichnen sich durch ihre hohe Wahrscheinlichkeit für photoelektrische Absorption im Energiebereich von einigen 10 keV bis 3 MeV aus.

Die andere Gruppe beinhaltet die Detektoren aus Materialien niedriger Ordnungszahl, deren Ordnungszahl $Z_{eff}$ kleiner gleich 30 ist. Hierzu gehören Plastik-Szintillatoren, organische Kristalle wie Anthracen, Stilben oder p-Terphenyl, reine und dotierte inorganische Kristalle wie z.B. $CaF_2$:Eu und das Halbleitermaterial Silizium. Die Materialien niedriger Ordnungszahl $Z_{eff} \leq 30$ besitzen eine hohe Wahrscheinlichkeit für Compton-Streuung im Energiebereich von 100 keV bis 3 MeV.

Aus diesen beiden Gruppen von Detektoren lassen sich zwei Typen von Detektorpaaren bilden, mit denen die Richtung der einfallenden Strahlung bestimmt wird. Es wird zwischen uni- und bidirektionalen Detektorpaaren unterschieden. Beide Typen von Detektorpaaren unterscheiden sich in Bezug auf die Richtung der gestreuten Strahlung.

Ein unidirektionales Detektorpaar enthält sowohl einen Detektor aus der Gruppe mit niedriger Ordnungszahl als auch einen Detektor aus der Gruppe mit mittlerer bis hoher Ordnungszahl.

Werden beispielsweise ein Plastik-Detektor und ein Cerbromid-Detektor miteinander kombiniert, ist das ein unidirektionales Detektorpaar. In einem unidirektionalen Detektorpaar hat jeder Detektor eine eindeutige physikalische Funktion. Wird ein Koinzidenzereignis mit der zum Nuklid gehörigen Strahlungsenergie E1+E2 beobachtet, ist zweifelsfrei feststellbar, dass die einfallende Strahlung zuerst im Plastik-Detektor gestreut wurde, bevor sie im Cerbromid-Detektor absorbiert wurde. Der umgekehrte Fall, dass die Strahlung zuerst im Cerbromid-Detektor gestreut wurde, bevor sie im Plastik-Detektor absorbiert wurde, hat eine Wahrscheinlichkeit nahe Null.

In einem unidirektionalen Detektorpaar ist die Richtung der *gestreuten* Strahlung bekannt. Die Strahlung wird im Detektor mit niedriger Ordnungszahl in Richtung auf den Detektor mit hoher Ordnungszahl gestreut.

Bidirektionale Detektorpaare bestehen aus der Kombination von je zwei Detektoren aus der Gruppe mit mittlerer und hoher Ordnungszahl, also z.B. von Cerbromid mit Cerbromid oder Germanium mit Germanium. Bei Verwendung von segmentierten Halbleiterdetektoren sind Paare aus je zwei Segmenten ebenfalls bidirektional, da beide Segmente aus dem gleichen Material bestehen.

In einem bidirektionalen Paar ist - insofern keine weiteren Informationen vorliegen - nicht feststellbar, welcher der beiden Detektoren die einfallende Strahlung zuerst gestreut hat. Die Richtung der *gestreuten* Strahlung bleibt unbekannt. Die Strahlung kann von Detektor 1 auf Detektor 2, aber auch von Detektor 2 auf Detektor 1 gestreut worden sein.

Es ist zu beachten, dass sowohl uni- als auch bidirektionale Detektorpaare in der Richtungsbestimmung bezüglich der *einfallenden* Strahlung beschränkt sind. Die Richtung der *einfallenden* Strahlung kann mit einem einzelnen Koinzidenzereignis nicht eindeutig rekonstruiert werden. Für den 2-dimensionalen Fall ist die Einfallsrichtung auf eine V-Linie, für den 3-dimensionalen Fall auf einen Kegelmantel festgelegt. Die Messdaten eines Einzelereignisses erlauben keine Aussage darüber, auf welchem Arm der V-Linie bzw. in welcher Richtung auf dem Kegelmantel die Strahlungsquelle liegt.

In Bezug auf die Richtungsrekonstruktion der *einfallenden* Strahlung stellen uni- und bidirektionale Detektorpaare gleichwertige Informationen bereit. Die Messdaten vieler Koinzidenzereignisse besitzen in beiden Fällen die für die Richtungsmessung erforderlichen statistischen Eigenschaften. Die Tatsache, dass die Richtung der *gestreuten* Strahlung in einem bidirektionalen Detektorpaar unbekannt ist, stellt keine experimentelle Einschränkung bezüglich der Richtungsbestimmung der *einfallenden* Strahlung dar.

Wie im Folgenden noch näher erläutert wird, hat die erfindungsgemäße Technik der richtungsaufgelösten Koinzidenz-Spektroskopie als wesentliches Herausstellungsmerkmal, dass sie sowohl für uni- als auch bidirektionale Detektorpaare nutzbar ist.

Die Detektoren eines Geräts können mehrfach auf unterschiedliche Paare aufgeteilt werden. Die Anzahl der Kombinationsmöglichkeiten wird durch die Detektormaterialien bestimmt. Detektoren aus der Gruppe mit niedriger Ordnungszahl lassen sich mit *jedem* Detektor aus der anderen Gruppe mit mittlerer oder hoher Ordnungszahl kombinieren. Detektoren aus der Gruppe mit mittlerer oder hoher Ordnungszahl können sogar mit *jedem* anderen Detektor in dem Gerät zu einem Paar zusammengestellt werden.

Die Gruppe der Detektoren mit niedriger Ordnungszahl ist zur Zusammenstellung gruppeninterner Paare hingegen nicht geeignet.

Ein Messprozess kann sowohl eine Kombination aus uni- und bidirektionalen Detektorpaaren nutzen oder ausschließlich unidirektionale oder ausschließlich bidirektionale Paare für die Richtungsmessung verwenden.

Nachdem die uni- und bidirektionalen Detektorpaare ausgewählt und mit einer Identifikationsnummer *i* versehen sind, werden die beiden Detektoren eines jedes Paares mit den Nummern 1 bzw. 2 gekennzeichnet. Enthält ein Paar unterschiedliche Detektormaterialien, so werden die Detektoren des Materials mit der niedrigeren Ordnungszahl als 1 bezeichnet, die Detektoren des Materials mit der höheren Ordnungszahl als 2. Wenn die Paare aus Detektoren des gleichen Materials bestehen, kann die Entscheidung, welcher Detektor in einem Paar als 1 bzw. 2 bezeichnet wird, willkürlich getroffen werden, insofern sie konsistent in der Datenauswertung beibehalten wird.

Im Folgenden wird davon ausgegangen, dass alle Strahlungsdetektoren für die Energiemessung kalibriert sind. Im Messprozess werden Koinzidenzereignisse akquiriert, die entsprechend ihrer Energiewerte E1 und E2 in den Detektoren 1 und 2 und der räumlichen Orientierung des Detektorpaars ein Tripel bilden. Die räumliche Orientierung wird über die Identifikationsnummer *i* des Detektorpaars abgebildet. Die beiden Energiewerte E1 und E2 enthalten die Informationen, die zum einen das Nuklid identifizieren, dessen Strahlung beobachtet wird, zum anderen aber auch die Richtung messen, aus der die Strahlung das Detektorpaar erreicht. Aufgrund der Energieerhaltung ist die Summe der registrierten Energieeinträge E1+E2 auf die Strahlungsenergie des Nuklids festgelegt. Beide Messwerte E1 und E2 sind stark miteinander korreliert und die Richtungsinformation ist wesentlich in der Differenz E2-E1 enthalten. Es ist daher möglich, die Anzahl der Variablen zu reduzieren, indem aus den beiden Energiewerten E1 und E2 ein neuer Messwert f(E1,E2) definiert wird, der die Richtungsinformation näherungsweise repräsentiert. Die zunächst als Tripel organisierten Messdaten (*i*, E1, E2) können so auf ein Dupel mit den zwei Merkmalen *i* und f(E1,E2) reduziert werden.

Da von stationären bzw. quasistationären Messbedingungen ausgegangen wird, können die Messdaten als 2-dimensionale Häufigkeitsverteilung $\underline{Y}$ gruppiert werden. Jedes Ereignis wird entsprechend seiner zwei Merkmale, *i* und f(E1,E2), in die Häufigkeitsverteilung $\underline{Y}$ einsortiert. Zur Erfassung der Messdaten wird für jedes Detektorpaar *i* ein Histogramm angelegt. Für die Histogramme ist der Wertebereich von f(E1,E2) in Klassen einzuteilen. Wird ein Koinzidenzereignis registriert, so kann aus den zwei Energiewerten E1 und E2 in den Detektoren 1 bzw. 2 ein geeigneter Funktionswert f(E1,E2) berechnet werden. Im Sinne der Erfindung wird die Funktion wie folgt definiert:

$$f(E1, E2) = A = \frac{E2 - E1}{E1 + E2} \qquad (1)$$

Bei Substitution von E2 mit C-E1 in Gl. (1) entsteht die Funktion f(E1) = 1 - 2E1/C, die auf dem gesamten Intervall [0,C] eindeutig definiert, stetig und monoton ist. Diese Funktion mit den genannten Eigenschaften ist sowohl für unidirektionale als auch für bidirektionale Detektorpaare geeignet. In Compton Kameras wird hingegen häufig der Compton Winkel $\vartheta$ verwendet,

$$\vartheta = \cos^{-1}\left(1 - \frac{mc^2}{E2} + \frac{mc^2}{E1 + E2}\right) \qquad (2)$$

mit $mc^2$ = 511 keV, der Ruheenergie des Elektrons.

Gl. (2) ist jedoch keine zulässige Funktion f(E1,E2) im Sinne der Erfindung, da $\vartheta(E1)$ nicht auf dem gesamten Intervall $0 \leq E1 \leq C$ eindeutig definiert ist.

Ein Koinzidenzereignis wird entsprechend seines Wertes f(E1,E2) in dasjenige Histogramm einsortiert, das zu dem Detektorpaar *i* gehört, in dem die Koinzidenz aufgetreten ist. Die Gesamtheit der Histogramme für alle Detektorpaare *i* = 1, ..., *I* bildet die 2-dimensionale Häufigkeitsverteilung $\underline{Y}$, die fortlaufend während einer Messung gefüllt wird.

Aus der aufgezeichneten Häufigkeitsverteilung $\underline{Y}$ wird nun die Richtungsverteilung $\underline{X}$ der Strahlungsquellen bestimmt. Entsprechend der experimentellen Situation werden zwei Fälle unterschieden. Bei der 3-dimensionalen Richtungsmessung wird die Richtungsverteilung als von zwei Variablen abhängige Funktion $X(\omega,h)$ auf der Himmelskugel modelliert. Die Himmelskugel ist eine in der Astronomie und in der Navigation gebräuchliche Kugel, mit der Positionen am Himmel bestimmt werden. Sie ist eine scheinbare, das Messgerät allseitig umgebende Kugel mit beliebig großem Radius, auf welche die Strahlungsquellen projiziert werden. Der Funktionswert $X(\omega, h)$ beschreibt die Strahlungsstärke in einem Raumwinkelsegment $d\Omega$ auf der Himmelskugel in der Azimutrichtung $\omega$ beim Höhenwinkel *h*.

Bei der 2-dimensionalen Richtungsmessung wird eine von einer Variablen abhängige Funktion $X(\omega)$ entlang des Horizontkreises betrachtet. Der Funktionswert $X(\omega)$ beschreibt die Strahlungsstärke in Richtung $\omega$. Es wird davon ausgegangen, dass die Quellen in der gleichen Ebene liegen, wie auch die Detektoren des Messgerätes.

Die Aufgabe des Algorithmus besteht darin, die Funktion $X(\omega, h)$ bzw. $X(\omega)$ zu bestimmen.

Die räumliche Richtungsverteilung $X(\omega, h)$ des Strahlungsfeldes (bzw. $X(\omega)$ im 2-dimensionalen Fall) kann nun mit beliebigen statistischen Bildrekonstruktionsverfahren, wie sie aus der Emissionstomographie bekannt sind, berechnet werden. Die statistischen Rekonstruktionsverfahren erlauben eine einheitliche Verarbeitung der Messdaten des gesamten Messgerätes, unabhängig davon, ob ein bestimmtes Detektorpaar uni- oder bidirektional ist.

Zu den gebräuchlichen statistischen Bildrekonstruktionsverfahren gehören u.a.

- Maximum Likelihood Expectation Maximization (MLEM) Algorithmus
- Ordered Subset Expectation Maximization (OSEM) Algorithmus
- List Mode - Maximum Likelihood Expectation Maximization (LM-MLEM) Algorithmus
- List Mode - Ordered Subset Expectation Maximization (LM-OSEM) Algorithmus

Alle genannten Verfahren sind Beispiele für Algorithmen, deren Anwendungsbereich nicht auf die Medizin beschränkt ist. Mit der zuvor beschriebenen Vorgehensweise gemäß dieser Erfindung sind sie ohne Weiteres auch im ABC- und Strahlenschutz einsetzbar.

Nachfolgend wird das erfindungsgemäße Verfahren an Hand von Ausführungsbeispielen und in Zeichnungen näher beschrieben. Die Erläuterungen sind lediglich beispielhaft und schränken den allgemeinen Erfindungsgedanken nicht ein.

Gemäß einem weiteren vorteilhaften Ausführungsbeispiel des erfindungsgemäßen Verfahrens emittieren die Strahlungsquellen eine diskrete und/oder kontinuierliche Verteilung von Strahlung.

Gemäß einem weiteren vorteilhaften Ausführungsbeispiel des erfindungsgemäßen Verfahrens emittieren die Strahlungsquellen neben Gammastrahlung auch Partikelstrahlung aus einer Partikelgruppe von Elektronen, Positronen, Protonen, Ionen- und/oder Neutronen.

Gemäß einem weiteren vorteilhaften Ausführungsbeispiel des erfindungsgemäßen Verfahrens stammt die Strahlung aus dem radioaktiven Zerfall eines oder mehrerer Radionuklide und/oder ist die Strahlung die prompte Gammastrahlung, welche bei der Absorption von Protonen- oder Ionenstrahlung in Targetmaterialien entsteht.

Gemäß einem weiteren vorteilhaften Ausführungsbeispiel des erfindungsgemäßen Verfahrens weist die Strahlung eine niedrige Intensität auf.

Gemäß einem vorteilhaften Ausführungsbeispiel des erfindungsgemäßen Verfahrens umfasst dieses in Schritt a) zusätzlich in Teilen oder in Gänze die folgende Abfolge von Schritten:

- **Kalibrieren** der Signale von allen Detektoren als absorbierte Strahlungsenergie E;

- **Festlegen** eines geeigneten Koordinatensystems;

- **Erfassen** der Richtungen der definierten Detektorpaare, wobei in der 2-dimensionalen Richtungsmessung die Richtung von Detektorpaar $i$ mit dem Azimutwinkel $\varphi_i$ erfasst wird und wobei in der 3-dimensionalen Richtungsmessung die Richtung von Detektorpaar $i$ mit dem Azimutwinkel $\varphi_i$ und dem Höhenwinkel $\beta_i$ erfasst wird;

- **Einteilen** des Messbereiches für den Funktionswert f(E1,E2) in eine Anzahl $J$ äquidistante Messwertkanäle $j = 1, ...,J$;

- **Anlegen** eines oder mehrerer 2-dimensionaler Arrays mit $I \cdot J$ Feldern als Datenstrukturen zur Speicherung der Häufigkeitsverteilungen $\underline{Y}$, in welchen Koinzidenzereignisse entsprechend ihrer ID-Nummer $i$ und ihres Messwertkanals $j$ registriert werden; für jedes Radionuklid wird ein separates 2-dimensionales Array $\underline{Y}$ angelegt;

- **Einteilen** der Detektorpaare $i$ in Symmetrieklassen $SK$, wobei diejenigen Detektorpaare $i$, die bei Drehung oder Verschiebung auf andere baugleiche Detektorpaare abgebildet werden, zu jeweils einer Symmetrieklasse $SK(i)$ zusammengefasst werden;

- **Anlegen** einer oder mehrerer Lookup-Tabellen $LUT^{SK}$ für jede Symmetrieklasse $SK$ und jedes Radionuklid, wobei diese einen Winkelbereich $\vartheta$ von 0° bis 180° umspannen und in äquidistante Winkelschritte eingeteilt werden;

- **Erstellen** der Lookup-Tabellen $LUT^{SK}$ durch Messungen mit dem Detektorsystem oder durch Monte Carlo Simulationen oder mittels eines theoretischen Modells;

- **Übergeben** sämtlicher Lookup-Tabellen $LUT^{SK}$ für alle Symmetrieklassen und alle Radionuklide an einen Algorithmus der Bildrekonstruktion, welcher die Messdaten verarbeitet und die Richtungsverteilungen $\underline{X}$ berechnet.

Vorzugsweise wird jeder Strahlungsdetektor eines Messgerätes für die Energiemessung kalibriert. Für Strahlungsdetektoren aus der Gruppe mit mittlerer bis hoher Ordnungszahl kann der photoelektrische Effekt zur Energiekalibration genutzt werden. Für solche Detektoren wird die Energiekalibration anhand der Photo-Peaks im Pulshöhen-Spektrum durchgeführt. Die Vorgehensweise ist den Experten auf dem Gebiet der Strahlungsdetektion wohl vertraut und soll hier nicht weiter erörtert werden.

Für Detektoren aus der Gruppe mit niedriger Ordnungszahl ist die Energiemessung jedoch weniger gebräuchlich, da diese häufig im Pulszähl-Modus betrieben werden. Es stehen mehrere Kalibrationsoptionen für Detektoren mit $Z_{eff} \leq 30$ zur Verfügung. Die Pulshöhen-Spektren dieser Detektoren können auf das Vorhandensein von Photo-Peaks untersucht werden. Wenn Photo-Peaks nachweisbar sind, können diese für die Energiekalibration genutzt werden. Wenn keine Photo-Peaks nachweisbar sind, stellt der Endpunkt des Compton Energiespektrums eine geeignete Alternative dar. Die maximale, in Compton Streuereignissen beobachtbare Energie $E_{C,max}$, die auch als Compton-Kante bezeichnet wird, ergibt sich zu

$$E_{C,max} = \frac{E_\gamma}{1 + \frac{mc^2}{2\,E_\gamma}} \tag{3}$$

aus der Strahlungsenergie $E_\gamma$ des jeweiligen Radionuklids, mit $mc^2 = 511$ keV, der Ruhenergie des Elektrons. Die Position der Compton-Kante lässt sich experimentell bestimmen, indem die erste Ableitung des Pulshöhen-Spektrums gebildet wird. Die Minimalstelle in der ersten Ableitung des Pulshöhen-Spektrums liefert in guter Näherung die Compton-Kante. Die Pulshöhe an der Minimalstelle kann dann gemäß Gl. (3) als Energie kalibriert werden.

Viele der gebräuchlichen Strahlungsdetektoren stellen keine Informationen über den Wechselwirkungspunkt der Strahlung im Detektormaterial bereit. Es ist daher erforderlich, eine Annahme über die Verbindungslinie zu treffen, welche die beiden Wechselwirkungspunkte in einem Detektorpaar miteinander verbindet. Eine naheliegende Annahme ist, hierfür die Linie zu wählen, welche beide Mittelpunkte der aktiven Detektormedien (des Szintillators oder des Halbleiters) verbindet. Für jedes Detektorpaar existiert dann eine Verbindungslinie, die durch die beiden Detektormittelpunkte verläuft.

Die Verbindungslinie eines Detektorpaars wird als gerichtete Größe betrachtet. Der Linie wird ein Vektor zugeordnet. Der Vektor hat seinen Ursprungsort im Mittelpunkt von Detektor 2 und zeigt auf den Mittelpunkt von Detektor 1.

Es sollen nun die Raumrichtungen der Detektorpaare festgelegt werden. Dabei wird von parallelem Strahlungseinfall auf das Messgerät ausgegangen (Fernfeld). Der Strahlungseinfall ist näherungsweise parallel, wenn die Abstände aller Strahlungsquellen sehr viel größer sind als die Abmaße des Messgerätes.

Zunächst ist ein geeignetes Koordinatensystem festzulegen, das sowohl für die Strahlungsquellen als auch für die Strahlungsdetektoren gilt. Bei der 3-dimensionalen Richtungsmessung ist dies ein Kugelkoordinatensystem, wobei der Koordinatenursprung im räumlichen Mittelpunkt der Detektoranordnung liegt. Im 2-dimensionalen Fall wird ein Polarkoordinatensystem verwendet.

Man stelle sich vor, dass das Koordinatensystem achsenparallel zu den kartesischen Koordinatenrichtungen im Raum verschoben werden kann. Für jedes Detektorpaar kann das Koordinatensystem so jeweils in den Mittelpunkt von Detektor 2 verschoben werden.

Betrachtet wird zunächst der 3-dimensionalen Fall: Die Kugelkoordinaten von Detektor 1 in diesem verschobenen Koordinatensystem, dessen Ursprung jeweils in Detektor 2 liegt, definieren einen charakteristischen Azimut- und Höhenwinkel für dieses Detektorpaar. Jedes Detektorpaar $i$ verfügt somit über einen solchen charakteristischen Azimutwinkel $\varphi_i$ und Höhenwinkel $\beta_i$. Beide mit dem Detektorpaar assoziierte Winkel $\varphi_i$ und $\beta_i$ werden in der Software hinterlegt. Sie werden in den Auswerteverfahren verwendet.

Im 2-dimensionalen Fall ist es ein Polarkoordinatensystem, das für jedes Detektorpaar $i$ achsenparallel zu den kartesischen Koordinatenrichtungen jeweils in den Mittelpunkt von Detektor 2 verschoben wird. Der Azimutwinkel $\varphi_i$ von Detektor 1 in diesem verschobenen Koordinatensystem wird für jedes Detektorpaar $i$ in einer Software hinterlegt.

Als weiterer Bestandteil nutzt das erfindungsgemäße Verfahren eine Systemmatrix, die das Ansprechverhalten des Gerätes auf eine punktförmige Strahlungsquelle beschreibt. Da der Rechenaufwand der Datenverarbeitung mit der Größe der Systemmatrix skaliert, ist es hilfreich, die Symmetrieeigenschaften des Messgerätes zu nutzen, um die Systemmatrix so klein wie möglich zu halten. Hierfür werden die Detektorpaare eines Messgerätes in Symmetrieklassen eingeteilt.

Diejenigen Detektorpaare, die bei Drehung oder Verschiebung auf andere baugleiche Detektorpaare abgebildet werden, gehören zu einer Symmetrieklasse. Sie werden zu einer Gruppe zusammengefasst und alle auf gleiche Weise behandelt. Die Systemmatrix ist als Gruppe von Lookup-Tabellen definiert. Jede Lookup-Tabelle beschreibt für jeweils eine Symmetrieklasse das Ansprechverhalten der Detektorpaare dieser Symmetrieklasse auf eine unendlich weit entfernte, punktförmige Strahlungsquelle. Die Lookup-Tabellen können anhand einer Simulationsrechnung, eines theoretischen Modells oder aus Messungen mit punktförmigen Referenzstrahlern gewonnen werden.

Zu jeder Symmetrieklasse und jedem Radionuklid gibt es eine Lookup-Tabelle. Die Gesamtheit aller Lookup-Tabellen bildet die Systemmatrix des Messgerätes. Die Lookup-Tabellen repräsentieren dabei die mathematischen Beziehungen zwischen den Strahlungsquellen und den Messdaten.

Die Elemente einer Lookup-Tabelle sind die Abbildungsfunktionen $LUT_j[\vartheta]$. Sie repräsentieren die Wahrscheinlichkeiten, dass eine Strahlungsquelle im Winkel $\vartheta$ zur Detektorpaarachse Koinzidenzereignisse in Bin $j$ der Messwertverteilung f(E1,E2) verursacht. Der Index $j$ indiziert die j-te Klasse der Messwertverteilung f(E1,E2).

Gemäß einem weiteren vorteilhaften Ausführungsbeispiel des erfindungsgemäßen Verfahrens werden in Schritt a) die Lookup-Tabellen $LUT^{SK}$ durch Messungen mit dem Detektorsystem erzeugt, indem die folgende Abfolge von Schritten ausgeführt wird:

- **Erstellen** einer Vorschrift zur Erzeugung und Validierung der Lookup-Tabellen $LUT^{SK}$ aus Referenzmessungen, welche die Referenzquellen auswählt, den Nuklidtyp berücksichtigt, sowie die Messbedingungen definiert, unter denen die Messungen durchzuführen sind;

- **Ausführen** von Referenzmessungen für alle in der Vorschrift zuvor definierten Schritte;

- **Erstellen** und **Validieren** der Lookup-Tabellen $LUT^{SK}$ gemäß der zuvor definierten Vorschrift zur Auswertung der Messdaten aus den Referenzmessungen;

- **Erfassen** des natürlichen Strahlungshintergrundes $b^{SK(i)}$ für alle Symmetrieklassen $SK$;

- **Exkludieren** des natürlichen Strahlungshintergrundes $b^{SK}$ aus den Lookup-Tabellen $LUT^{SK}$;

Im Folgenden wird eine Ausführungsform der Erfindung beschrieben, wie die Lookup-Tabellen experimentell mit Hilfe von punktförmigen Referenzstrahlern generiert werden. Alle Referenzstrahler sollen weit genug vom Messgerät entfernt sein, dass die Strahlung quasi parallel auf das Gerät fällt (Fernfeld).

Es ist von Vorteil, die Messungen mit mehreren Referenzquellen unterschiedlicher Nuklide durchzuführen. Die Quellen sollen danach ausgewählt werden, dass sie den Energiebereich repräsentieren, in dem das Messgerät eingesetzt wird. Der Abstand der Quellen soll groß genug sein, um parallelen Strahlungseinfall zu gewährleisten. Idealerweise wird der Abstand so groß wie möglich gewählt, wie es die Stärke der Quelle erlaubt, um Messdaten in sinnvollen Akquisitionszeiten zu gewinnen.

Für jede Quelle wird eine Serie mehrerer Messungen an unterschiedlichen Winkelpositionen ausgeführt. Es ist darauf zu achten, dass die Quelle in allen Messungen immer den gleichen radialen Abstand zum Gerätemittelpunkt hat. Alle Messungen werden mit gleicher Messzeit t ausgeführt. Die Datenakquisition soll unter gleichen Bedingungen erfolgen, wie sie auch für den operativen Betrieb vorgesehen sind.

Wenn die Einsatzbestimmungen des Messgerätes vorsehen, nicht nur relative Intensitäten, sondern auch absolute Strahlungsintensitäten in Maßeinheiten von z.B. pGy sr$^{-1}$ s$^{-1}$ oder nSv sr$^{-1}$ h$^{-1}$ bereitzustellen, ist darauf zu achten, dass ausschließlich kalibrierte Referenzstrahler zum Einsatz kommen.

In allen anderen Fällen genügt es, wenn die Daten der Lookup-Tabelle bei konstanter relativer Strahlungsstärke akquiriert werden; eine absolute Kalibration der Strahlungsintensität ist dann nicht erforderlich. Es können dann Quellen beliebiger Stärke verwendet werden, insofern ihre Aktivität für eine gute Zählerstatistik ausreicht.

Nach Abschluss der Messungen mit den Referenzquellen sollte in einer weiteren Messung ohne Quelle der natürliche Strahlungshintergrund aufgenommen werden. Die Untergrundmessung soll bei gleicher Messdauer t ausgeführt werden wie zuvor die Messungen mit den Referenzquellen.

Die Vorgehensweise zur Gewinnung der Lookup-Tabellen aus den Messdaten ist für alle Nuklide gleich und wird hier exemplarisch für ein Nuklid beschrieben.

Es wird davon ausgegangen, dass alle Detektorpaare in Symmetrieklassen eingeteilt sind. Jede Symmetrieklasse hat eine Identifikationsnummer $SK(i)$, die für jedes Detektorpaar $i$ zu definieren ist.

Für jede Symmetrieklasse $SK$ wird eine separate Lookup-Tabelle $LUT^{SK}$ angelegt, die den Winkelbereich von 0° bis 180° umspannt und in äquidistante Winkelschritte eingeteilt wird.

Die an einer bestimmten Winkelposition aufgezeichneten Daten sind für jedes Detektorpaar auszuwerten. Entsprechend der aktuellen Position der Referenzquelle bei einem Azimutwinkel $\omega$ und einem Höhenwinkel $h$ wird ein Winkelabstand $\vartheta_i$ für jedes Detektorpaar $i$ berechnet:

$$\vartheta_i = \arccos\left(\cos\beta_i \cos h \cos(\omega - \varphi_i) + \sin\beta_i \sin h\right) \qquad (4)$$

Im obigen Ausdruck bezeichnet $\varphi_i$ den Azimutwinkel und $\beta_i$ den Höhenwinkel von Detektorpaar *i*. Im 2-dimensionalen Fall kann der Winkelabstand $\vartheta_i$ mit

$$\vartheta_i = \arccos\left(\cos(\omega - \varphi_i)\right) \qquad (5)$$

berechnet werden.

Der Vorgang wird für alle Winkelpositionen der Referenzquelle wiederholt.

Die Messdaten werden nun entsprechend der Zugehörigkeit der Detektorpaare zu Symmetrieklassen geordnet, um daraus die Lookup-Tabellen zu erzeugen. Für alle Paare *i* einer Symmetrieklasse *SK(i)* liegen die Messwerte bei den Winkelabständen $\vartheta_i$ vor. Das Ziel ist, für jede Symmetrieklasse *SK* die zugehörige Lookup-Tabelle $LUT^{SK}$ im gesamten Wertebereich von $\vartheta = 0\,°$ bis $180\,°$ mit Daten zu füllen.

Für jeden Wert $\vartheta$ aus dem Bereich von $\vartheta = 0\,°$ bis $180\,°$ wird nun jeweils der Datensatz $\vartheta_i$ einer Symmetrieklasse *SK* ausgesucht, der dem aktuellen Wert $\vartheta$ am nächsten liegt. Dieser Datensatz wird an die Stelle $\vartheta$ der Lookup-Tabelle kopiert. Man verfahre auf diese Weise, bis alle Stellen $\vartheta$ von 0° bis 180° einer Lookup-Tabelle mit Daten gefüllt sind. Der Vorgang ist für alle Symmetrieklassen durchzuführen. Entsprechend dieser Vorgehensweise werden die Lookup-Tabellen eines Messgeräts generiert.

Die Messung ohne Referenzquelle dient dazu, den natürlichen Strahlungshintergrund zu messen. Der natürliche Strahlungshintergrund kann als isotrop angenommen werden. Alle Untergrund-Datensätze, die zur gleichen Symmetrieklasse gehören, können gemittelt werden. Der gemittelte Datensatz wird mit $b_j^{SK}$ bezeichnet und gilt für alle Detektorpaare einer Symmetrieklasse *SK.*

Der in den Lookup-Tabellen enthaltene Strahlungshintergrund kann herausgerechnet werden, indem von jedem Element der Lookup-Tabelle $LUT_j^{SK}(\vartheta)$ der Untergrund $b_j^{SK}$ im Bin *j* der Messwertverteilung f(E1,E2) subtrahiert wird.

Ein zentraler Bestandteil des erfindungsgemäßen Verfahrens sind die aufgezeichneten Lookup-Tabellen. Lookup-Tabellen sind energiespezifisch und gelten jeweils für die charakteristische Strahlungsenergie der Strahlungsquelle. Zur Einrichtung eines Messgeräts für bestimmte Einsatzbedingungen sind verschiedene Optionen möglich. Ist die Einsatzbestimmung an eine überschaubare Anzahl von Nukliden gekoppelt, kann es sinnvoll sein, für jedes Nuklid einen Satz von Lookup-Tabellen anzulegen. Wenn das Messgerät hingegen in einem breiten Energiebereich mit ganz unterschiedlichen Nukliden verwendet werden soll, ist es vorteilhaft, eine weitere Stufe der Datenaufbereitung durchzuführen. Die Lookup-Tabellen können mit Funktionen gefittet werden. Die Fitparameter werden für verschiedene Gammaenergien erfasst und als energieabhängige Funktionen hinterlegt. Während einer Messung können aus den gespeicherten energieabhängigen Funktionen Lookup-Tabellen für beliebige Gammaenergien erzeugt werden. Das Gerät kann so dynamisch an die konkret vorliegenden Situationen angepasst werden. Es können Strahlungsquellen über einen weiten Energiebereich abgebildet werden.

Gemäß einem weiteren vorteilhaften Ausführungsbeispiel des erfindungsgemäßen Verfahrens wird in Schritt e) eine Selektionsbedingung bezüglich der Energiesumme E1+E2 der in beiden Detektoren eines Paares detektierten Energien angewendet. In Ausführungsbeispielen, wenn mehrere Radionuklide vorliegen, werden die Selektionsbedingungen genutzt, um in Schritt e) für jedes Radionuklid eine separate Häufigkeitsverteilung **_Y_** anzulegen und/oder in Schritt f) für jedes Radionuklid eine separate Richtungsverteilung **_X_** zu berechnen.

Bei der Erfassung der 2-dimensionalen Häufigkeitsverteilung **_Y_** ist es empfehlenswert, eine Auswahl der Koinzidenzereignisse mit einer Selektionsbedingung für die Energiesumme E1+E2 vorzunehmen. Es sollen nur solche Werte f(E1,E2) in die Verteilung **_Y_** einsortiert werden, deren Energiesumme E1+E2 innerhalb eines vordefinierten Bereichs um den Energiewert liegt, welcher der charakteristischen Nuklidenergie entspricht, dessen Strahlung detektiert wird. Mit Hilfe einer solchen Selektionsbedingung ist es möglich, separate Häufigkeitstabellen für jedes Nuklid auszuwerten.

Gemäß einem weiteren vorteilhaften Ausführungsbeispiel des erfindungsgemäßen Verfahrens werden in Schritt e) bei Radionukliden mit mehreren Gammaenergien mehrere Selektionsbedingungen bezüglich der Energiesumme E1+E2 angewendet und/oder für solche Radionuklide mit mehreren Gammaenergien werden eine oder mehrere Häufigkeitsverteilungen **_Y_** angelegt.

Werden die Richtungen von Quellen eines Nuklids gemessen, das Strahlung auf mehreren Linien emittiert, liegt es im Ermessen des Anwenders, ob die Koinzidenzereignisse in einer oder in mehreren Häufigkeitsverteilungen erfasst werden sollen. Beispielsweise besitzt Co-60 zwei nah beieinander liegende Linien bei 1173 keV und 1332 keV. Koinzidenzereignisse beider Emissionen können ohne Probleme in die gleiche Häufigkeitsverteilung einsortiert werden.

Da beide Emissionslinien so nah beieinander liegen, sind auch die 2-dimensionalen Häufigkeitsverteilungen sehr ähnlich und können ohne Schwierigkeiten zusammen ausgewertet werden. Hat jedoch ein Nuklid mehrere, weit auseinanderliegende Gammalinien ist es empfehlenswert, diese getrennt zu erfassen. Die zu einem Nuklid gehörigen Häufigkeitsverteilungen werden später in dem Auswerteverfahren wieder zusammengeführt.

Gemäß einem weiteren vorteilhaften Ausführungsbeispiel des erfindungsgemäßen Verfahrens ist das statistische Bildrekonstruktionsverfahren in Schritt f) ein Teil von oder in Gänze das Maximum Likelihood Expectation Maximization (MLEM) Verfahren, das Ordered Subset Expectation Maximization (OSEM) Verfahren, das List Mode - Maximum Likelihood Expectation Maximization (LM-MLEM) Verfahren und/oder das List Mode - Ordered Subset Expectation Maximization (LM-OSEM) Verfahren.

Im Folgenden werden zwei Ausführungsformen der Erfindung beschrieben, wie eine räumliche Richtungsverteilung $X(\omega, h)$ bzw. eine ebene Richtungsverteilung $X(\omega)$ mit dem Maximum Likelihood Expectation Maximization (MLEM) Algorithmus berechnet werden können. Die Ausführungen gelten für Geräte, in denen die Summe $\sum_{i,j} Y_{ij}$ aller Einträge in der Häufigkeitsverteilung $\underline{Y}$ keine Richtungsabhängigkeit aufweist. Bei Vorhandensein vieler Detektorpaare mittelt sich die Richtungsabhängigkeit individueller Detektorpaare heraus; die Gesamtzählrate $\sum_{i,j} Y_{ij}$ ist dann näherungsweise richtungsunabhängig.

Gemäß einem weiteren vorteilhaften Ausführungsbeispiel des erfindungsgemäßen Verfahrens wird in Schritt f) für jedes detektierte Radionuklid eine 3-dimensionale Richtungsverteilung $X_{kl} = X(\omega_k, h_l)$ gemäß

$$X_{kl}^{[n+1]} = X_{kl}^{[n]} \frac{KL}{\sum_{i,j} Y_{ij}} \sum_{i,j} \frac{LUT_j^{SK(i)}[\vartheta(\omega_k, h_l, \varphi_i, \beta_i)] \, Y_{ij}}{\sum_{k'=1}^{K} \sum_{l'=1}^{L} LUT_j^{SK(i)}[\vartheta(\omega_{k'}, h_{l'}, \varphi_i, \beta_i)] \, X_{k'l'}^{[n]}}$$

aus einer Anfangsverteilung

$$\forall \, k = 1, \dots, K, l = 1, \dots, L \qquad X_{kl}^{[0]} = 1$$

unter Nutzung von Lookup-Tabellen $LUT^{SK}$ und einer Winkelabstandsfunktion gemäß

$$\vartheta(\omega_k, h_l, \varphi_i, \beta_i) = \arccos \, (\cos \beta_i \cos h_l \cos(\omega_k - \varphi_i) + \sin \beta_i \sin h_l)$$

berechnet, wobei K und L vom Nutzer definierte Anzahlen an Pixeln für den Azimutwinkel $\omega_k$ und den Höhenwinkel $h_l$ sind.

Nachfolgend wird ein Algorithmus zur Rekonstruktion einer 3-dimensionalen Richtungsverteilung beschrieben. Die Oberfläche der Himmelskugel wird zunächst in Breiten- und Längengrade eingeteilt. Jedes der gleich großen Flächenelemente auf der Himmelskugel entspricht einem Bildpixel *(k,l)* für das die Bestrahlungsstärke $X_{kl} = X(\omega_k, h_l)$ beim Azimutwinkel $\omega_k$ und Höhenwinkel $h_l$ berechnet werden soll. Man achte darauf, dass die Klassen für den Höhenwinkel $h_l$ so gewählt werden, dass der Sinus des Höhenwinkels gleichverteilt im Intervall (-1,1) ist. Es gibt insgesamt K·L Pixel $X_{kl}$ für K Klassen $\omega_k$ und L Klassen $h_l$.

Maximum Likelihood Expectation Maximization (MLEM) ist ein iteratives Verfahren, das Anfangswerte für die Richtungsverteilung $\underline{X}$ benötigt:

$$\forall \, k = 1, \dots, K, l = 1, \dots, L \qquad X_{kl}^{[0]} = 1 \qquad\qquad (6)$$

Die MLEM Iterationsvorschrift zur Berechnung einer neuen Darstellung $X_{kl}^{[n+1]}$ aus den Vorgängerwerten $X_{kl}^{[n]}$ lautet:

$$X_{kl}^{[n+1]} = X_{kl}^{[n]} \frac{KL}{\sum_{i,j} Y_{ij}} \sum_{i,j} \frac{LUT_j^{SK(i)}[\vartheta(\omega_k, h_l, \varphi_i, \beta_i)] \, Y_{ij}}{\sum_{k'=1}^{K} \sum_{l'=1}^{L} LUT_j^{SK(i)}[\vartheta(\omega_{k'}, h_{l'}, \varphi_i, \beta_i)] \, X_{k'l'}^{[n]}} \qquad (7)$$

Hierin bezeichnet $Y_{ij}$ die in der Häufigkeitstabelle $\underline{Y}$ registrierten Zähler (die Messwerte) für ein Detektorpaar *i* im Bin *j* der Messwertverteilung f(E1,E2). Beim Aufruf der Lookup-Tabellen $Lur^{SK(i)}$ ist der Winkelabstand $\vartheta$ zwischen einem Pixel *(k,l)* auf der Himmelskugel und einem Detektorpaar *i* zu berechnen. Im 3-dimensionalen Fall kann der Winkelabstand $\vartheta$ gemäß

$$\vartheta(\omega_k, h_l, \varphi_i, \beta_i) = \arccos\ (\cos\beta_i \cos h_l \cos(\omega_k - \varphi_i) + \sin\beta_i \sin h_l) \qquad (8)$$

berechnet werden, wobei $\varphi_i$ der Azimutwinkel und $\beta_i$ der Höhenwinkel von Detektorpaar $i$ sind.

Der MLEM Algorithmus ist ein Verfahren, das eine Häufigkeitsverteilung, nämlich die gemessene Häufigkeitsverteilung $\underline{Y}$, die angibt wie häufig in bestimmten Detektorpaaren bei bestimmten Messwerten Koinzidenzen aufgetreten sind, verwendet und daraus eine neue Häufigkeitsverteilung berechnet, nämlich die gesuchte Richtungsverteilung $\underline{X}$, die angibt, mit welcher Häufigkeit die Strahlung aus bestimmten Einfallswinkeln $\omega_k$ und $h_l$ am Detektionsort eintrifft.

Gleichung (7) ist so formuliert, dass die Iterationsvorschrift parallel zur Datenakquisition ausgeführt werden kann, während noch fortlaufend Koinzidenz-Zähler $Y_{ij}$ in der Häufigkeitsverteilung $\underline{Y}$ akkumuliert werden.

Gemäß einem weiteren vorteilhaften Ausführungsbeispiel des erfindungsgemäßen Verfahrens wird in Schritt f) für jedes detektierte Radionuklid eine 2-dimensionale Richtungsverteilung $X_k = X(\omega_k)$ gemäß

$$X_k^{[n+1]} = X_k^{[n]} \frac{K}{\sum_{i,j} Y_{ij}} \sum_{i,j} \frac{LUT_j^{SK(i)}[\vartheta(\omega_k, \varphi_i)]\ Y_{ij}}{\sum_{k'=1}^{K} LUT_j^{SK(i)}[\vartheta(\omega_{k'}, \varphi_i)]\ X_{k'}^{[n]}}$$

aus einer Anfangsverteilung

$$\forall\, k = 1, \dots, K \qquad X_k^{[0]} = 1$$

unter Nutzung von Lookup-Tabellen $LUT^{SK}$ und einer Winkelabstandsfunktion gemäß

$$\vartheta(\omega_k, \varphi_i) = \arccos\ (\cos(\omega_k - \varphi_i))$$

berechnet, wobei K eine vom Nutzer definierte Anzahl an Pixeln für den Azimutwinkel $\omega_k$ ist.

In einer weiteren Ausführung der Erfindung kann eine ebene Richtungsverteilung $X_k = X(\omega_k)$ mit dem MLEM Algorithmus berechnet werden. Es wird eine Anfangsverteilung initialisiert

$$\forall\, k = 1, \dots, K \qquad X_k^{[0]} = 1 \qquad (9)$$

und eine Iterationsschleife mit der Iterationsvorschrift:

$$X_k^{[n+1]} = X_k^{[n]} \frac{K}{\sum_{i,j} Y_{ij}} \sum_{i,j} \frac{LUT_j^{SK(i)}[\vartheta(\omega_k, \varphi_i)]\ Y_{ij}}{\sum_{k'=1}^{K} LUT_j^{SK(i)}[\vartheta(\omega_{k'}, \varphi_i)]\ X_{k'}^{[n]}} \qquad (10)$$

durchlaufen. Der Winkelabstand $\vartheta$ wird im 2-dimensionalen Fall mit

$$\vartheta(\omega_k, \varphi_i) = \arccos\ (\cos(\omega_k - \varphi_i)) \qquad (11)$$

berechnet.

Die Richtungsverteilung $\underline{X}$ des Strahlungsfeldes ist zunächst einheitenlos. Die Werte $X_{kl}$ repräsentieren die relativen Strahlungsintensitäten in den verschiedenen Einfallsrichtungen $\omega_k$ und $h_l$. Es ist aber auch möglich, die Strahlungsintensität mit der Maßeinheit einer Dosisleistung pro Raumwinkel anzugeben. Dafür ist es notwendig, das Detektorsystem zunächst so einzurichten, dass nuklidspezifische Dosisleistungen gemessen werden können.

Zur Messung nuklidspezifischer Dosisleistungen werden Zählraten von Wechselwirkungsereignissen verwendet, deren Gesamtenergie der jeweiligen Nuklidenergie entspricht. Es gibt dabei vielfältige Optionen. Zum einen können die nuklidspezifischen Zählraten von allen im Gerät enthaltenen Detektoren mittlerer bis hoher Ordnungszahl verwendet werden, andererseits können auch Zählraten von Koinzidenzereignissen genutzt werden, deren Gesamtenergie E1+E2 in einem bestimmten Intervall um die Nuklidenergie liegt. Diese Zählraten können in Einheiten der Äquivalenz-Dosisleistung oder in Einheiten der absorbierten Dosisleistung (in Luft) kalibriert werden. Die Vorgehensweise soll hier kurz dargelegt werden.

Die Kalibration wird mit einer Referenzquelle kalibrierter Aktivität $\Lambda$ durchgeführt. Die Dosisleistung $\dot{D}$ bzw. die Äquivalenz-Dosisleistung $\dot{H}^*(10)$ am Gerätemittelpunkt können aus der Aktivität $\Lambda$ der Quelle, ihrem Abstand r und den

nuklidspezifischen Dosisleistungskonstanten $\Gamma$ bzw. $\Gamma_H$ berechnet werden. Die (physikalische) Dosisleistung $\dot{D}$ ergibt sich aus:

$$\dot{D} = \frac{dD}{dt} = \frac{\Lambda\,\Gamma}{r^2} \tag{12}$$

und die Äquivalenz-Dosisleistung $\dot{H}^*(10)$ aus:

$$\dot{H}^*(10) = \frac{dH^*(10)}{dt} = \frac{\Lambda\,\Gamma_H}{r^2} \tag{13}$$

Die Werte der Dosisleistungskonstante $\Gamma$ und der Äquivalenz-Dosisleistungskonstante $\Gamma_H$ für die verschiedenen Nuklide können in Tabellen der einschlägigen Fachliteratur aufgefunden werden.

Von den zur Verfügung stehenden nuklidspezifischen Zählraten des Gerätes ist nun eine Zählrate oder eine Kombination von Zählraten auszuwählen, die unter Vermeidung systematischer Fehler geeignet ist, die Dosisleistung zu repräsentieren. Eine mögliche Ursache für systematische Fehler bei der Messung der Dosisleistung ist ein ungleichmäßiges Ansprechverhalten des Gerätes in Bezug auf die Richtung der einfallenden Strahlung. Die Zählraten können beispielsweise durch Verschattungseffekte beeinträchtigt sein. Insbesondere die Koinzidenz-Zählraten zeichnen sich durch ihre starke Richtungsabhängigkeit aus. Die für die Richtungsmessung vorteilhafte Richtungsempfindlichkeit der Koinzidenz-Ereignisse kann eine erhebliche Fehlerursache bei der Messung der Dosisleistung sein.

Nachfolgend werden zwei Ausführungen der Erfindung vorgestellt, wie eine Messung nuklidspezifischer Dosisleistungen mit kleinen systematischen Fehlern möglich ist.

Für Geräte, bei denen Verschattungseffekte vernachlässigbar sind, ist es sinnvoll, die in den Detektoren $m = 1, ..., M$ hoher Ordnungszahl detektierten nuklidspezifischen Zähler $N_m$ im Photo-Peak zu betrachten. Die Zählrate $\dot{Z}$, definiert als:

$$\dot{Z} = \frac{d\big(\mathrm{Max}(N_1, ..., N_M)\big)}{dt} \tag{14}$$

kann ein gutes Maß für die Dosisleistung sein, wenn es in jeder Richtung mindestens einen Detektor hoher Ordnungszahl gibt, der der einfallenden Strahlung ungehindert ausgesetzt ist.

Eine alternative Vorgehensweise bietet sich für Geräte an, die aus vielen Detektoren bestehen. Hier kann die Summe aller Koinzidenz-Zähler in der Häufigkeitsverteilung $\underline{Y}$ als Maß für die absorbierte Dosis dienen, vorausgesetzt, dass die Gesamtzählrate

$$\dot{Z} = \frac{d\big(\sum_{i,j} Y_{ij}\big)}{dt} \tag{15}$$

richtungsunabhängig ist.

Die Eignung einer bestimmten Variable $\dot{Z}$ zur Kalibration der Dosisleistung lässt sich überprüfen, indem eine Referenzquelle bei konstantem Abstand r um das Gerät bewegt wird. Bleibt die Zählrate $\dot{Z}$ konstant, während die Quelle um das Gerät bewegt wird, ist die Bedingung erfüllt. Die Variable $\dot{Z}$ kann dann zur Kalibration der Dosisleistung verwendet werden.

Für die Kalibration der Dosisleistung mit der Zählrate $\dot{Z}$ wird eine Untergrundmessung ohne Quelle aufgezeichnet. Die so ermittelte Zählrate wird als $\dot{Z}_b$ bezeichnet. Es können nun gerätespezifische Kalibrationskonstanten $C$ und $C_H$ definiert werden, mit denen die Dosisleistung $\dot{D}$

$$\dot{D} = C\,(\dot{Z} - \dot{Z}_b) \tag{16}$$

und die Äquivalenz-Dosisleistung $\dot{H}^*(10)$

$$\dot{H}^*(10) = C_H\,(\dot{Z} - \dot{Z}_b) \tag{17}$$

aus der untergrundkorrigierten Zählrate $\dot{Z} - \dot{Z}_b$ berechnet werden können.

Anhand der gerätespezifischen Kalibrationskonstanten $C$ und $C_H$ können nun richtungsabhängige Dosisleistungen pro Raumwinkel berechnet werden. Hierfür werden die Intensitätswerte $X_{kl}$ mit einem Skalierungsfaktor multipliziert. Im Folgenden wird vorausgesetzt, dass die Zählrate $\dot{Z}$ gemäß Gl. (15) und die Kalibrationskonstanten $C$ und $C_H$ gemäß Gl. (16) bzw. (17) definiert werden.

Die Dosisleistung $\dot{D}$ pro Raumwinkel $d\Omega$ wird mit

$$\frac{dD(\omega_k, h_l)}{dt\, d\Omega} = \frac{C\,(\dot{Z} - \dot{Z}_b)}{4\pi}\, X_{kl} \tag{18}$$

die Äquivalenz-Dosisleistung $\dot{H}\,^*(10)$ pro Raumwinkel $d\Omega$ mit:

$$\frac{dH^*(10)\,(\omega_k, h_l)}{dt\, d\Omega} = \frac{C_H\,(\dot{Z} - \dot{Z}_b)}{4\pi}\, X_{kl} \tag{19}$$

berechnet.

Im 2-dimensionalen Fall gilt:

$$\frac{dD(\omega_k)}{dt\, d\omega} = \frac{C\,(\dot{Z} - \dot{Z}_b)}{2\pi}\, X_k \tag{20}$$

und:

$$\frac{dH^*(10)\,(\omega_k)}{dt\, d\omega} = \frac{C_H\,(\dot{Z} - \dot{Z}_b)}{2\pi}\, X_k \tag{21}$$

Für den Dosisleistungsbereich bis zu 1 $\mu$Sv/h kann die (physikalische) Dosisleistung pro Raumwinkel z.B. mit der Maßeinheit pGy sr$^{-1}$ s$^{-1}$ angegeben werden. Die Äquivalenz-Dosisleistung pro Raumwinkel kann beispielsweise in Einheiten von nSv sr$^{-1}$ h$^{-1}$ gemessen werden.

Wird die Dosisleistung pro Raumwinkel über alle Einfallsrichtungen gemittelt, ergibt sich die Dosisleistung $\dot{D}$, welche aus allen Richtungen empfangen wird, dividiert durch $4\pi$. Gleiches gilt für die Äquivalenz-Dosisleistung: Die über alle Einfallsrichtungen gemittelte Äquivalenz-Dosisleistung pro Raumwinkel ist gleich der Äquivalenz-Dosisleistung $\dot{H}\,^*$ (10), dividiert durch $4\pi$.

Die Erfindung betrifft weiter eine Vorrichtung zur Ausführung des erfindungsgemäßen Verfahrens. Wobei die erfindungsgemäße Vorrichtung hierbei eine Gruppe von mehreren synchronisierten Detektoren zur Erfassung von Strahlung umfasst, wobei mindestens ein Detektormaterial eine Ordnungszahl von $Z_{eff} > 30$ aufweist und alle Detektoren die Energien E messen, welche in Wechselwirkungen der Strahlung mit den Detektormaterialien auftreten.

Weiter weist die erfindungsgemäße Vorrichtung eine Systemelektronik auf, welche Koinzidenzereignisse registriert, wenn in je zwei Detektoren aus einer Liste von definierten Detektorpaaren $i$ gleichzeitig Wechselwirkungen stattfinden, wobei die Liste von definierten Detektorpaaren alle Paare umfasst die kombinatorisch aus der Menge aller Detektoren gebildet werden können, und die definierten Detektorpaare mindestens einen Detektor aus einem Material mit einer Ordnungszahl von $Z_{eff} > 30$ enthalten.

Weiter weist die erfindungsgemäße Vorrichtung ein Datenakquisitionssystem auf, das für beide an einem Koinzidenzereignis beteiligte Detektoren eine Rangordnung festlegt, die einen ersten und einen zweiten Detektor definiert, und die in Koinzidenzereignissen gemessenen Energien (E1,E2) entsprechend ihrer Kennzeichnung 1,2 ordnet und in einer chronologischen Liste mit den Attributen { $i$, E1, E2 } und der Detektionszeit $t$ speichert; wobei in jedem Detektorpaar $i$ der Detektor mit der niedrigeren Ordnungszahl die Nummer 1 und derjenige mit der höheren Ordnungszahl die Nummer 2 erhält; sollten beide Detektoren eines Paares die gleiche Ordnungszahl haben, wird die Kennzeichnung in 1 bzw. 2 willkürlich getroffen.

Darüber hinaus weist die erfindungsgemäße Vorrichtung eine Analyseeinheit auf, die für jedes Koinzidenzereignis aus den vom Datenakquisitionssystem gespeicherten Energien (E1,E2) einen Funktionswert f(E1,E2) = (E2-E1) / (E1+E2) berechnet und die Koinzidenzereignisse entsprechend ihrer Detektorpaarnummer $i$ und ihrer Funktionswerte f(E1,E2) in einer oder mehreren Häufigkeitsverteilungen $\underline{Y}$ erfasst und mit einem statistischen Bildrekonstruktionsverfahren der Emissionstomographie unter Nutzung von Lookup-Tabellen $LUT^{SK}$ aus jeder Häufigkeitsverteilung $\underline{Y}$ je eine Richtungsverteilung $\underline{X}$ des Strahlungsfeldes berechnet, wobei für jedes Radionuklid je eine Häufigkeitsverteilung $\underline{Y}$ und je eine Richtungsverteilung $\underline{X}$ vorliegen.

Gemäß einem vorteilhaften Ausführungsbeispiel der erfindungsgemäßen Vorrichtung ist diese teilweise oder komplett als eine Compton Kamera, ein Compton Teleskop, eine Single Plane Compton Kamera, eine Neutronenkamera und/oder eine duale Gamma/Neutronenkamera ausgebildet.

Im Sinne der Erfindung wird unter einer Vorrichtung auch ein Messgerät, kurz Gerät oder ein Detektorsystem verstanden.

Gemäß einem weiteren vorteilhaften Ausführungsbeispiel der erfindungsgemäßen Vorrichtung hat die Gesamtheit aller Detektoren der Vorrichtung eine ringförmige Gestalt, bei der die Detektoren in einem Ring angeordnet sind und/oder wobei im Inneren des Rings kein, ein oder mehrere Zentraldetektoren vorliegen. Weiter enthält der Ring bevorzugt vier oder fünf und besonders bevorzugt sechs Plastik-Szintillationsdetektoren. Weiter befinden sich im Inneren des Rings bevorzugt ein oder zwei Szintillationsdetektoren aus NaI, CsI, CeBr$_3$ und/oder LaBr$_3$.

Gemäß einem weiteren vorteilhaften Ausführungsbeispiel der erfindungsgemäßen Vorrichtung ist diese bevorzugt aus Szintillationsdetektoren der Größe 1"x1", 1,5"x1,5", 2"x2" und/oder 3"x3" aufgebaut.

Gemäß einem weiteren vorteilhaften Ausführungsbeispiel der erfindungsgemäßen Vorrichtung wird als Detektor ein Szintillationsdetektor verwendet und/oder ist der Szintillator als monolithischer Block oder als pixeliertes Szintillatormodul ausgebildet und/oder welcher aus reinen oder dotierten Materialien der Gruppe von PVT, Anthracen, Stilben, p-Terphenyl, CaF$_2$, BaF$_2$, NaI, CeBr$_3$, LaBr$_3$, LaCl$_3$, La(Br$_x$Cl$_{1-x}$)$_3$, CsI, SrI$_2$, CLYC, CLBC, CLCB, CLLB, BGO, LSO, LYSO, GAGG, YAP und/oder YAG gebildet ist.

Gemäß einem weiteren vorteilhaften Ausführungsbeispiel der erfindungsgemäßen Vorrichtung wird als Detektor ein Halbleiterdetektor verwendet und/oder ist der Halbleiter als segmentierter oder unsegmentierter Halbleiter ausgebildet und/oder welche eine planare oder koaxiale Geometrie aufweist und/oder welcher aus Materialien der Gruppe von Ge, GaAs, CdTe und/oder CdZnTe gebildet ist.

Gemäß einem weiteren vorteilhaften Ausführungsbeispiel der erfindungsgemäßen Vorrichtung werden mind. zwei Detektoren verwendet.

Gemäß einem weiteren vorteilhaften Ausführungsbeispiel der erfindungsgemäßen Vorrichtung sind alle verwendeten Detektoren im Wesentlichen baugleich sind oder mindestens zwei der verwendeten Detektoren sind voneinander verschieden.

Gemäß einem weiteren vorteilhaften Ausführungsbeispiel der erfindungsgemäßen Vorrichtung wird eine Detektor- und Systemelektronik verwendet, die analoge und/oder digitale Elektronikkomponenten benutzt. Dabei können die analogen Elektronikkomponenten eine Kombination verschiedener Module umfassen, zu denen eine Hochspannungsversorgung, ein Vorverstärker, ein Verstärker, ein Pulsformer, ein Ladungsintegrator, ein Pulshöhenanalysator, ein Multikanalanalysator (MCA) und/oder eine Koinzidenzschaltung zählen.

Gemäß einem weiteren vorteilhaften Ausführungsbeispiel der erfindungsgemäßen Vorrichtung können die digitalen Elektronikkomponenten eine Kombination verschiedener Hard- und Softwarekomponenten umfassen, zu denen eine Hochspannungsversorgung, ein A/D-Wandler pro Detektor, ein Field Programmable Gate Array (FPGA), ein Speichermedium, ein digitaler Signalprozessor und/oder eine Auswertesoftware zählen.

Es zeigen:

Abb. 1 eine nicht-maßstabsgetreue, schematische Darstellung eines unidirektionalen Detektorpaars in einer zweidimensionalen Messsituation, gemäß einer Ausführungsform der Erfindung;

Abb. 2 eine nicht-maßstabsgetreue, schematische Darstellung eines bidirektionalen Detektorpaars in einer zweidimensionalen Messsituation gemäß einer weiteren Ausführungsform der Erfindung;

Abb. 3 eine nicht-maßstabsgetreue, schematische Darstellung des Strahlungseinfalls von einer Quelle auf ein Detektorpaar 1,2 in einer zwei-dimensionalen Messsituation, gemäß einer weiteren Ausführungsform der Erfindung;

**Abb. 4a eine** nicht-maßstabsgetreue, schematische Darstellung eines Vorrichtungsmodells mit einem zentralen Cerbromid-Detektor der von sechs ringförmig angeordneten Plastik-Detektoren umgeben ist; gemäß einer weiteren Ausführungsform der Erfindung;

Abb. 4b eine nicht-maßstabsgetreue, schematische Darstellung eines Vorrichtungsmodelles gemäß Abb. 4a im dem ein weiterer Cerbromid-Detektor eingebracht ist, gemäß einer weiteren Ausführungsform der Erfindung;

Abb. 5 eine schematische Darstellung einer Häufigkeitsverteilung $\underline{Y}$ des Vorrichtungsmodells aus Abb. 4a in einer 2-dimensionalen Richtungsmessung, gemäß einer weiteren Ausführungsform der Erfindung;

Abb. 6a eine nicht-maßstabsgetreue, schematische Darstellung einer ersten Symmetrieklasse von insgesamt vier zu einem Quadrat angeordneten, würfelförmigen Detektoren, wobei zwei horizontale und zwei vertikale Detektorpaare vorliegen, gemäß einer weiteren Ausführungsform der Erfindung;

Abb. 6b eine nicht-maßstabsgetreue, schematische Darstellung einer weiteren Symmetrieklasse der vier zu einem Quadrat angeordneten, würfelförmigen Detektoren aus Abb. 6a, gemäß einer weiteren Ausführungsform der Erfindung;

Abb. 7 eine schematische Darstellung einer Häufigkeitsverteilung $\underline{Y}$ des Vorrichtungsmodells aus Abb. 4b in einer 3-dimensionalen Richtungsmessung, gemäß einer weiteren Ausführungsform der Erfindung;

Abb. 8a eine nicht-maßstabsgetreue, schematische Darstellung einer Richtungsverteilung für ein Strahlungsfernfeld, dessen Emissionspunkte alle in einer Ebene liegen. Die Richtungsverteilung wird als vom Azimutwinkel $\omega$ abhängige Funktion $X(\omega)$ entlang des Horizontkreises modelliert, gemäß einer weiteren Ausführungsform der Erfindung;

Abb. 8b eine nicht-maßstabsgetreue, schematische Darstellung einer Richtungsverteilung für ein Strahlungsfernfeld, dessen Emissionspunkte beliebig im Raum verteilt sind. Die Richtungsverteilung wird als vom Azimutwinkel $\omega$ und vom Höhenwinkel $h$ abhängige Funktion $X(\omega, h)$ auf der Himmelskugel modelliert, gemäß einer weiteren Ausführungsform der Erfindung;

Abb. 9 eine schematische Darstellung einer Lookup-Tabelle, gemäß einer weiteren Ausführungsform der Erfindung;

**Abb. 10 ein** Diagramm mit gemessenen Abbildungsfunktionen aus einer Lookup-Tabelle des Vorrichtungsmodells aus Abb. 4a für $CeBr_3$ - Plastik Detektorpaare bei ausgewählten Asymmetriewerten A; gemäß einer weiteren Ausführungsform der Erfindung;

Abb. 11 vier Diagramme von berechneten Asymmetriekurven in den Lookup-Tabellen von uni- und bidirektionalen Detektorpaaren für die Radionuklide Co-60 und Cs-137, gemäß einer weiteren Ausführungsform der Erfindung;

Abb. 12 eine nicht-maßstabsgetreue, schematische Darstellung eines Messplans mit Winkelpositionen einer Referenzquelle zur Erstellung einer Lookup-Tabelle für das Vorrichtungsmodell aus Abb. 4a , gemäß einer weiteren Ausführungsform der Erfindung;

Abb. 13 eine schematische Darstellung eines Rechenschemas für ein MLEM Verfahren zur Rekonstruktion der Richtungsverteilung eines Strahlungsfeldes, gemäß einer weiteren Ausführungsform der Erfindung;

Abb. 14 neun Diagramme von gemessenen Richtungsverteilungen für Strahlungsfelder mit einer, zwei und drei punktförmigen Strahlungsquellen. Die Darstellungen sind Beispiele für 2-dimensionale Richtungsmessungen mit dem Vorrichtungsmodell aus Abb. 4a, gemäß einer weiteren Ausführungsform der Erfindung;

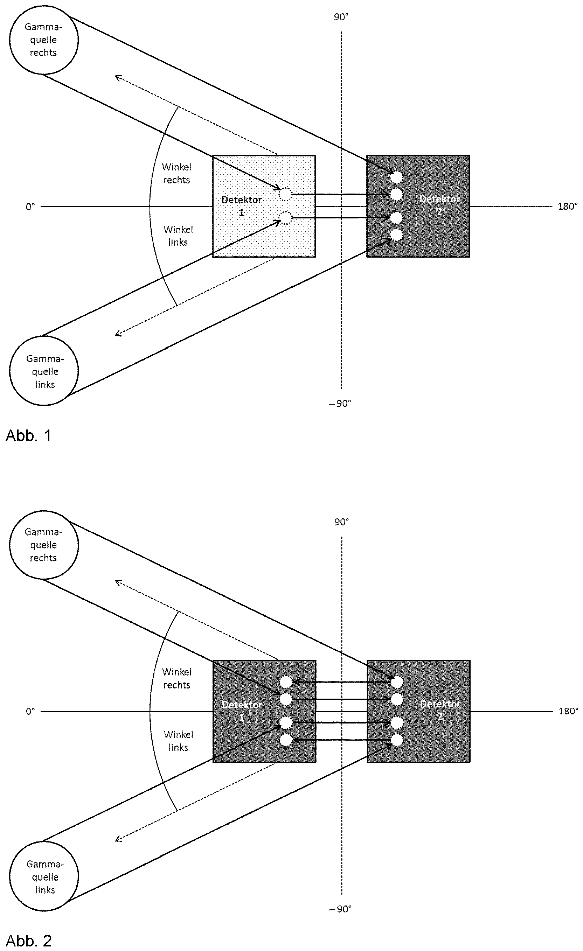

In der Abb. 1 ist eine nicht-maßstabsgetreue, schematische Darstellung eines unidirektionalen Detektorpaares in einer zwei-dimensionalen Messsituation dargestellt. Der Detektor mit Ordnungszahl $Z_{eff} \leq 30$ ist mit der Nummer 1 gekennzeichnet, der Detektor mit höherer Ordnungszahl $Z_{eff} > 30$ mit der Nummer 2. Die Rekonstruktion der Einfallsrichtung bleibt zweideutig. Zwar kann der Winkel der einfallenden Strahlung zur Verbindungslinie beider Detektoren gemessen werden, nicht jedoch die Seite, auf welcher die Quelle liegt.

Abb. 2 zeigt eine nicht-maßstabsgetreue, schematische Darstellung eines bidirektionalen Detektorpaares in einer zwei-dimensionalen Messsituation. Beide Detektoren bestehen aus Materialien mit gleicher oder ähnlicher Ordnungszahl $Z_{eff} > 30$. Die Kennzeichnung der zwei Detektoren eines bidirektionalen Paares in Detektor 1 und Detektor 2 wird einmal festgelegt und ist dann in allen Berechnungen konsistent anzuwenden. Wie auch bei dem unidirektionalen Detektorpaar in Abb. 1 bleibt die Rekonstruktion der Einfallsrichtung zweideutig. Es kann der Winkel der einfallenden Strahlung zur Verbindungslinie beider Detektoren gemessen werden, nicht jedoch die Seite, auf welcher die Quelle liegt.

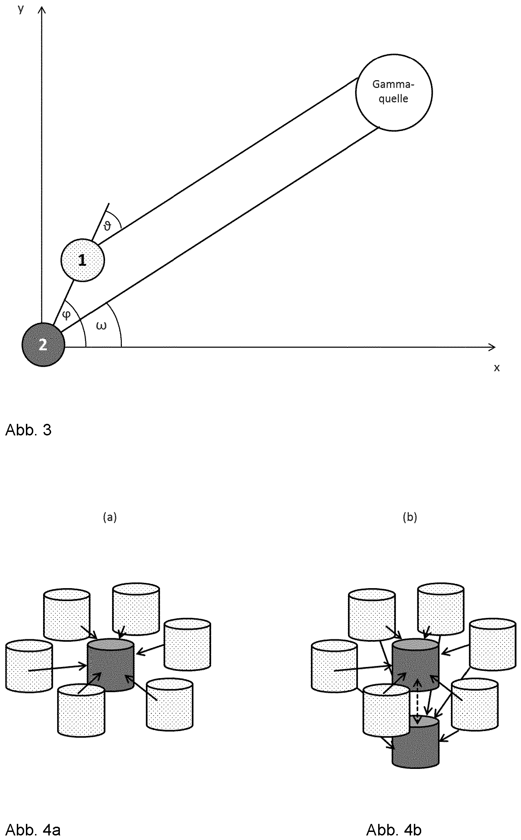

Abb. 3 zeigt den Strahlungseinfall einer Quelle auf ein Detektorpaar 1,2 in einer zwei-dimensionalen Messsituation.

Abb. 4 zeigt zwei Vorrichtungsmodelle gemäß der Erfindung. Im Modell von Abb. 4a ist ein zentraler Cerbromid-Detektor von sechs ringförmig angeordneten Plastik-Detektoren umgeben. Dieses Modell ist für die 2-dimensionale Richtungsmessung geeignet. Wird unter dem zentralen Cerbromid-Detektor ein weiterer Cerbromid-Detektor eingebracht, entsteht das Modell von Abb. 4b, welches 3-dimensionale Richtungsmessungen erlaubt.

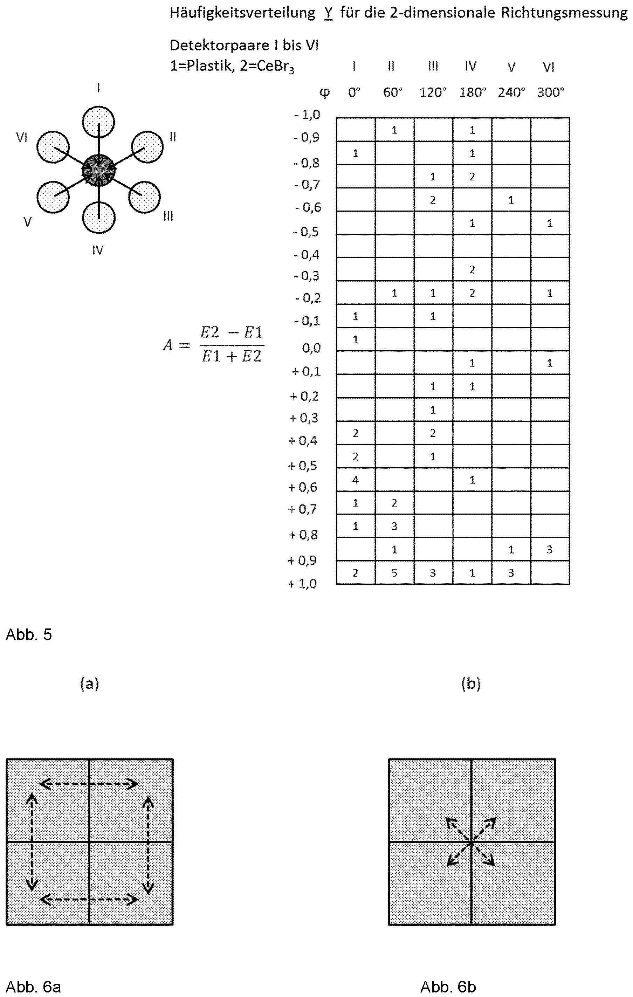

Abb. 5 zeigt ein Beispiel für eine Häufigkeitsverteilung $\underline{Y}$ (die Messdaten) für das Vorrichtungsmodell aus Abb. 4a. Die Systemelektronik stellt fest, welche Detektoren koinzident waren und berechnet den Asymmetriewert $A = (E2 - E1)/(E1 + E2)$ aus den koinzidenten Energieeinträgen E1 und E2. Die Koinzidenzereignisse werden entsprechend ihrer Detektorpaar-Identifikationsnummer und ihres Asymmetriewertes $A$ in der Häufigkeitsverteilung $\underline{Y}$ registriert.

Abb. 6 veranschaulicht wie Detektorpaare in Symmetrieklassen eingeteilt werden. Vier würfelförmige Detektoren sind in einem Quadrat angeordnet. Diese Detektoranordnung besitzt zwei Symmetrieklassen. Die erste Symmetrieklasse, in Abb. 6a gezeigt, umfasst zwei horizontale und zwei vertikale Detektorpaare. Die beiden diagonalen Detektorpaare, in Abb. 6b zu sehen, bilden eine weitere Symmetrieklasse.

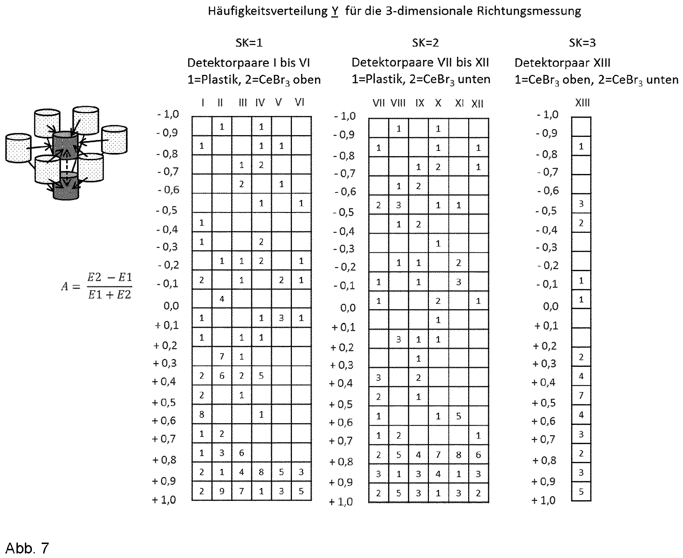

Abb. 7 zeigt ein Beispiel für eine Häufigkeitsverteilung $\underline{Y}$ (die Messdaten) für das Vorrichtungsmodell aus Abb. 4b. Die Messdaten $\underline{Y}$ wurden entsprechend der drei Symmetrieklassen des Vorrichtungsmodells aus Abb. 4b in drei Bereiche gruppiert. Detektorpaare I bis VI gehören zur Symmetrieklasse 1, Detektorpaare VII bis XII zur Symmetrieklasse 2. Das dreizehnte Detektorpaar bildet eine eigene Symmetrieklasse 3.

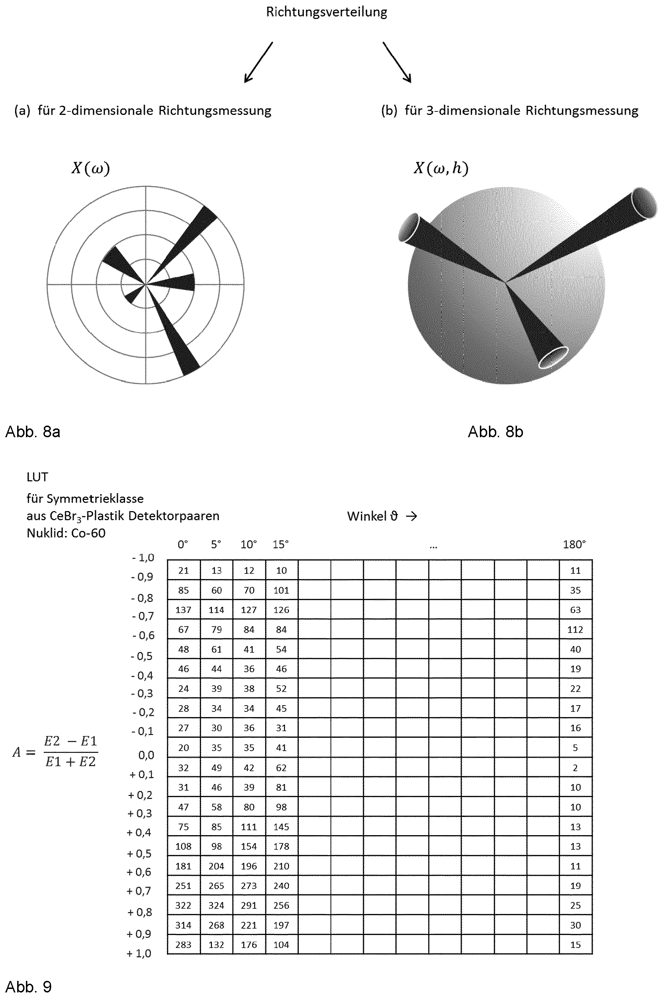

Abb. 8 zeigt zwei schematische Darstellungen von Richtungsverteilungen für Strahlungsfernfelder. Ein Strahlungsfernfeld ist durch seine Richtungsabhängigkeit vollständig charakterisiert. In Abhängigkeit von der Messsituation gibt es zwei Sorten von Verteilungen. Wenn alle Strahlungsquellen und das Messgerät in einer Ebene liegen, wird die vom Azimutwinkel $\omega$ abhängige Verteilung $X(\omega)$ entlang des Horizontkreises verwendet, in Abb. 8a gezeigt. Die allgemeingültige Richtungsverteilung ist die in Abb. 8b dargestellte, vom Azimutwinkel $\omega$ und vom Höhenwinkel $h$ abhängige Verteilung $X(\omega, h)$ auf der Himmelskugel. Die Größe $X$ repräsentiert die Strahlungsintensität. Sie kann entweder auf den größten vorkommenden Wert normiert werden oder mit einer Maßeinheit angegeben werden.

Abb. 9 zeigt schematisch eine Lookup-Tabelle. Eine Lookup-Tabelle ist eine 2-dimensionale Häufigkeitsverteilung, die Koinzidenzzähler als Funktion des Winkelabstands $\vartheta$ einer Strahlungsquelle von der Detektorpaarachse und einer Messgröße erfasst. Die Messgröße ist hier die Energieasymmetrie $A = (E2 - E1)/(E1 + E2)$ der beiden Energieeinträge in einem Koinzidenzereignis.

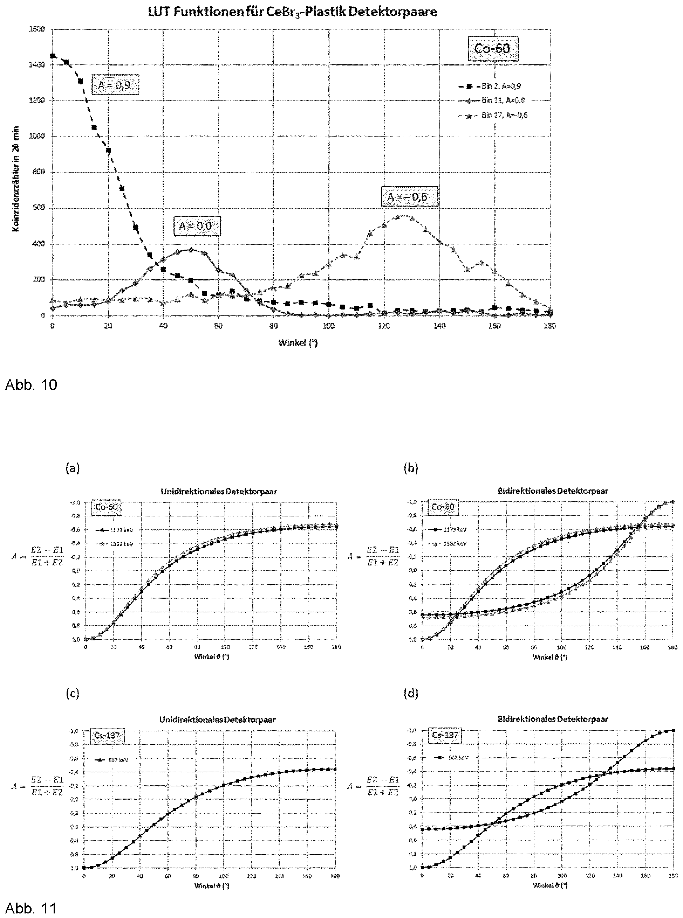

Abb. 10 zeigt verschiedene gemessene Abbildungsfunktionen aus einer Lookup-Tabelle für $CeBr_3$-Plastik Detektorpaare bei ausgewählten Asymmetriewerten $A$. Die Abbildungsfunktionen repräsentieren die Häufigkeiten, dass eine im Winkel $\vartheta$ einfallende Strahlung in bestimmten Messwertintervallen von A registriert wird. Eine Lookup-Tabelle kann als Gruppe von Abbildungsfunktionen aufgefasst werden. Das Beispiel zeigt drei der mit einer 10 µCi Co-60 Quelle aufgezeichneten Funktionen. Es wurden Koinzidenzereignisse beider Co-60 Emissionslinien bei 1173 keV und 1332 keV zusammen gespeichert.

Abb. 11 zeigt Asymmetriekurven für Lookup-Tabellen, die für die Radionuklide Co-60 und Cs-137 berechnet wurden, gemäß einer Ausführung der Erfindung. Die Asymmetriekurven zeigen die Bereiche in einer Lookup-Tabelle mit den höchsten Häufigkeiten. Gäbe es keine Messfehler, würden alle von Null verschiedenen Häufigkeiten auf den gezeigten Kurven liegen. Es wird hier nach Lookup-Tabellen von uni- und bidirektionalen Detektorpaaren differenziert. Lookup-Tabellen unidirektionaler Detektorpaare (Abb. 11 a und c) zeichnen sich dadurch aus, dass es nur eine markante Asymmetriekurve gibt. Hingegen besitzen Lookup-Tabellen bidirektionaler Detektorpaare (Abb. 11 b und d) zwei markante Asymmetriekurven. Für Co-60 sind die Asymmetriekurven für beide Emissionslinien bei 1173 keV und 1332 keV dargestellt; Cs-137 besitzt nur eine Emissionslinie bei 662 keV.

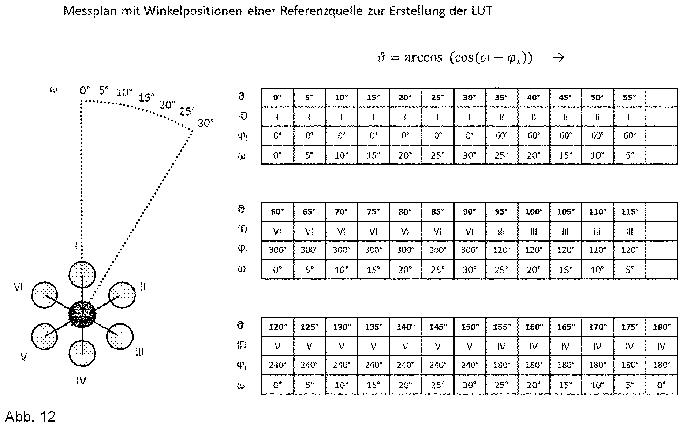

Abb. 12 zeigt beispielhaft die Winkelpositionen $\omega$ eines Messplans zur Erstellung der Lookup-Tabelle für das in Abb. 4a gezeigte Vorrichtungsmodell gemäß einer Ausführung der Erfindung. Nach diesem Plan werden Messungen in sieben Winkelstellungen durchgeführt. Die Quelle hat dabei immer den gleichen Abstand zum Gerätemittelpunkt. Aus den sieben Messungen kann eine Lookup-Tabelle für das Vorrichtungsmodell erzeugt werden. In diesem Beispiel hat die Lookup-Tabelle eine Schrittweite von 5°. Zu jedem Winkel $\vartheta$ im Wertebereich von 0° bis 180° gibt es mindestens ein Detektorpaar, das den korrekten Winkelabstand zur Strahlungsquelle besitzt und dessen Datensatz an die jeweilige Stelle in der Lookup-Tabelle kopiert werden kann. Der Messplan aus Abb. 12 definiert die Zuordnungen zwischen den Winkelpositionen $\omega$ der Referenzquelle, den Detektorpaaren und den Winkelwerten $\vartheta$ der Lookup-Tabelle.

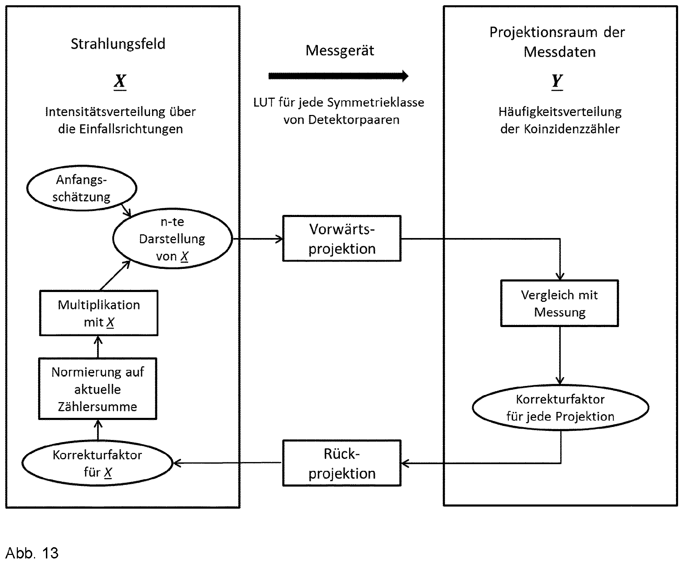

Abb. 13 zeigt ein Rechenschema für ein MLEM Verfahren zur Rekonstruktion der Richtungsverteilung eines Strahlungsfeldes gemäß einer Ausführung der Erfindung. Mit jedem Iterationsschritt n werden alle in Abb. 13 gezeigten Rechenschritte ausgeführt. Mit fortschreitender Anzahl n an Iterationen führt das MLEM Verfahren zu der Richtungsverteilung $\underline{X}$, welche die höchste Wahrscheinlichkeit besitzt, die beobachtete Häufigkeitsverteilung $\underline{Y}$ verursacht zu haben. Die Gleichungen (13) und (16) beinhalten sämtliche in Abb. 13 dargestellten Rechenschritte in kompakter Form, einschließlich der Vorwärts- und Rückprojektion.

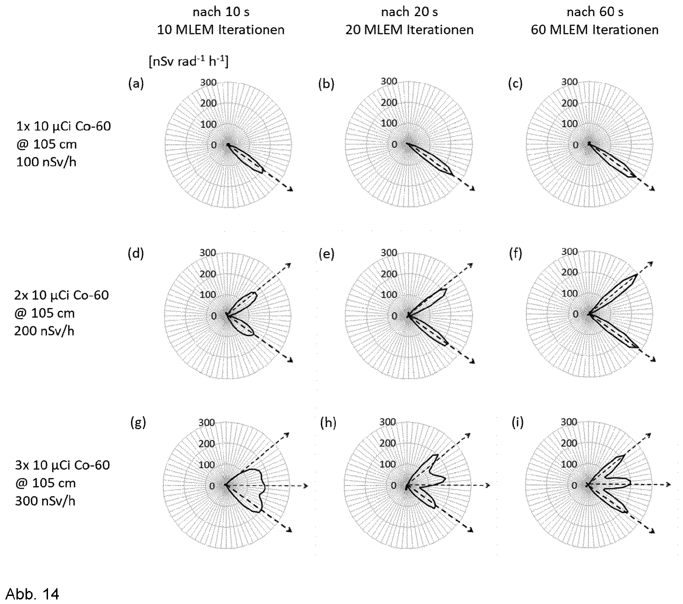

Abb. 14 zeigt verschiedene Beispiele für 2-dimensionale Richtungsmessungen mit dem Vorrichtungsmodell von Abb. 4a. Alle Messungen wurden mit je 10 µCi Co-60 Quellen in je 105 cm Abstand durchgeführt. Die radiale Messwert-Skala ist in allen Darstellungen gleich und erstreckt sich von 0 bis 300 nSv rad$^{-1}$ h$^{-1}$. Abb. 14a, 14b und 14c zeigen Messergebnisse für eine Co-60 Quelle bei einem Einfallswinkel von 125°. Anschließend wurden sukzessive eine zweite Co-60 Quelle bei 50° und schließlich eine dritte Co-60 Quelle bei 90° hinzugefügt. Abb. 14d, 14e und 14f zeigen die gemessenen Richtungsverteilungen für das 2-Quellen Strahlungsfeld, Abb. 14g, 14h und 14i für das 3-Quellen Strahlungsfeld.

Das erfindungsgemäße Verfahren sowie die Vorrichtung verwenden uni- und bidirektionale Detektionsprozesse zur mehrdimensionalen Richtungsmessung von Gammastrahlung. Die Richtungsmessung, welche teilweise oder vollständig von bidirektionalen Detektionsprozessen Gebrauch macht, wurde in den zuvor genannten Ausführungsbeispielen detailliert beschrieben. Der bidirektionalen Richtungsmessung liegt das in Abb. 11 dargestellte Funktionsprinzip zu Grunde, das hier kurz erläutert werden soll.

Im Unterschied zu unidirektionalen Detektionsprozessen, die einen funktionalen Zusammenhang zwischen einem Messwert (hier die Asymmetrie A) und dem Streuwinkel $\vartheta$ für die Richtungsmessung nutzen, besitzen bidirektionale Detektionsprozesse keine solche Kalibrationskurve $A(\vartheta)$. Die Lookup-Tabellen bidirektionaler Detektorpaare zeigen zwei markante Strukturen entlang der in Abb. 11b und 11d gezeigten Asymmetriekurven. Zu einem bestimmten Streuwinkel $\vartheta$ gibt es jeweils zwei zugehörige Messwerte $A1$ und $A2$, die mit bestimmten Häufigkeiten beobachtet werden. Dennoch kann dem Wertepaar ($A1,A2$) eindeutig ein bestimmter Streuwinkel $\vartheta$ zugeordnet werden. Über den gesamten Winkelbereich von 0° bis 180° gibt es jedes Wertepaar ($A1,A2$) genau einmal. Es kann mit keinem anderen Wertepaar ($A1$, $A2$) bei einem anderen Winkel $\vartheta$ verwechselt werden. Es ist daher auch mit einem bidirektionalen Detektorpaar möglich, den Streuwinkel $\vartheta$ zu messen. Statistische Rekonstruktionsverfahren verfügen bereits über die zur Richtungsmessung mit bidirektionalen Detektorpaaren erforderliche Funktionalität. Verfahren wie MLEM sind daher für die Verarbeitung der Messdaten aus den erfindungsgemäßen Vorrichtungsmodellen geeignet.

Es sollen hier Messungen mit dem Vorrichtungsmodell aus Abb. 4a vorgestellt werden, das aus sechs 3"×3" Plastik-Szintillationsdetektoren und einem 3"×3" Cerbromid-Szintillationsdetektor aufgebaut ist. Die sechs Plastik-Detektoren bilden einen Ring um den zentralen Cerbromid-Detektor. Der Durchmesser des Detektorrings beträgt 25 cm.

Jeder der sechs Plastik-Detektoren kann mit dem zentralen Cerbromid-Detektor zu einem unidirektionalen Detektorpaar kombiniert werden. In einem solchen Detektorpaar wird die Strahlung vornehmlich im Plastik-Detektor gestreut und im Cerbromid-Detektor absorbiert.

Abb. 14 zeigt gemessene Richtungsverteilungen für verschiedene Anordnungen aus mehreren punktförmigen Strahlungsquellen. Alle Messungen wurden mit je 10 $\mu$Ci Co-60 Quellen in je 105 cm Abstand durchgeführt. Zunächst wurde eine erste Co-60 Quelle bei einem Winkel von 125° platziert. Die von einer 10 $\mu$Ci Co-60 Quelle in 105 cm Abstand erzeugte Dosisleistung beträgt ca. 100 nSv h$^{-1}$. Abb. 14 a), b) und c) zeigen die Ergebnisse der Richtungsmessung nach jeweils 10 s, 20 s und 60 s Messzeit. Die Zahl der MLEM Iterationen wurde dynamisch an die Messzeit gekoppelt. Mit fortschreitender Messdauer wird die Richtungsverteilung der Punktquelle steiler und höher. Die über alle Richtungen gemittelte Intensität liegt konstant bei 16 nSv rad$^{-1}$ h$^{-1}$. Integriert über den Vollwinkel von $2\pi$ rad ergibt sich eine Dosisleistung von $2\pi$ rad $\cdot$ 16 nSv rad$^{-1}$ h$^{-1}$ = 100 nSv h$^{-1}$. Anschließend wurde eine zweite Co-60 Quelle bei 50° hinzugefügt. Die Ergebnisse sind in Abb. 14 d), e) und f) zu sehen. Das Vorhandensein zweier Quellen ist bereits ab 10 s Messzeit deutlich erkennbar. Die über alle Richtungen gemittelte Intensität liegt jetzt bei 32 nSv rad$^{-1}$ h$^{-1}$. Zuletzt wurde der Anordnung eine dritte Co-60 Quelle bei 90° hinzugefügt. Die Ergebnisse in Abb. 14 g), h) und i) zeigen, dass ab 20 s Messzeit auch drei Punktquellen separat aufgelöst werden können.

Es wurde eine Methodik entwickelt, mit der die Güte der Richtungsmessung als Zeitangabe formuliert werden kann, ab der eine verlässliche Richtungsmessung vorliegt. Das Ziel der Vorgehensweise war, die Güte der Richtungsmessung einer Punktquelle in Abhängigkeit vom Nuklidtyp, der Dosisleistung und der Winkelposition quantitativ zu bewerten. Als Quellen standen je eine Co-60, eine Cs-137 und eine Co-57 Quelle der Aktivität von je 10 $\mu$Ci zur Verfügung.

Es wurden zwei Güteklassen definiert: $\pm5°$ und $\pm10°$ Winkelgenauigkeit. Zu jedem Zeitpunkt $t$ während einer Messung liefert das MLEM Verfahren einen Schätzwert für die Richtung der Strahlungsquelle. Die Messungen liefen jeweils über eine bestimmte Messdauer und wurden 20 Mal wiederholt. Zu jedem Zeitpunkt t ist nun feststellbar, wieviele der Messungen in die $\pm5°$ oder $\pm10°$ Güteklasse fallen. Es wurde ein Konfidenzlevel von 90% gewählt. $t_{90}$ ist als diejenige Zeit definiert, ab der mindestens 18 der insgesamt 20 Messungen richtig sind, d.h. in einem $\pm5°$ oder $\pm10°$ Intervall um den wahren Wert liegen. In 90% aller Messungen wird die angegebene Genauigkeit nach der Mindestmesszeit erreicht oder übertroffen.

Das Vorrichtungsmodell von Abb. 4a besitzt eine charakteristische Symmetrie. Zuerst wurde untersucht, welchen Einfluss die gerätespezifische Detektoranordnung auf die Mindestmesszeiten hat, welche bei verschiedenen Einfallswinkeln gemessen werden. Die Empfindlichkeit der Richtungsmessung hängt von der Lage der Quelle relativ zum Gerät ab. Liegt die Quelle in Richtung eines der sechs Ringdetektoren werden längere Messzeiten benötigt, als wenn die Quelle in der Mitte zwischen zwei Ringdetektoren liegt. In dieser Untersuchung hatten alle Quellen einen konstanten Abstand von 1 m zum Gerätemittelpunkt. Die Mindestmesszeit $t_{90}$ für $\pm10°$ Genauigkeit der 10 $\mu$Ci Co-60 Quelle betrug 2,7 s in Richtung der Ringdetektoren und verkürzte sich auf 0,5 s in der Mitte zwischen zwei Ringdetektoren. Ein ähnliches Verhalten wurde für Cs-137 beobachtet. Hier betrug die Mindestmesszeit $t_{90}$ für $\pm10°$ Genauigkeit 5,8 s in Richtung der Ringdetektoren und verkürzte sich auf 0,7 s in der Mitte zwischen zwei Ringdetektoren.

Als nächstes wurde untersucht, wie die $t_{90}$ Mindestmesszeiten von der Dosisleistung abhängen. Diese Messungen wurden jeweils in Richtung der Ringdetektoren durchgeführt. Sie stellen die untere Grenze der Leistungsfähigkeit des Gerätes dar. Der Abstand der Quellen wurde von 1 bis 5 Meter variiert. Die Dosisleistung wurde als Ortsdosisleistung $\dot{H}*(10)$ der Quelle am Messpunkt ausgedrückt. Dabei wurde festgestellt, dass oberhalb von 0,05 $\mu$Sv/h das Produkt aus Ortsdosisleistung $\dot{H}*(10)$ und $t_{90}$ Mindestmesszeit konstant ist.

Unter dieser Voraussetzung können die $t_{90}$ Mindestmesszeiten mit dem einfachen analytischen Ausdruck

$$t_{90} = T(\text{Nuklid}, \sigma) \cdot \left( \frac{\dot{H}^*(10)}{\mu Sv/h} \right)^{-1} \qquad (22)$$

parametrisiert werden. Die Parameter $T$(Nuklid, $\sigma$) sind gerätespezifische Parameter, die in Abhängigkeit vom Nuklidtyp und der Güteklasse $\sigma$ definiert werden. Für das Vorrichtungsmodell mit sechs 3"x3" Plastik-Detektoren und einem 3"x3" Cerbromid-Detektor wurden die Parameter folgendermaßen bestimmt: $T$($^{60}$Co, $\pm 10°$) = 0,32 s, $T$($^{60}$Co, $\pm 5°$) = 0,48 s, $T$ ($^{137}$Cs, $\pm 10°$) = 0,16 s und $T$($^{137}$Cs, $\pm 5°$) = 0,24 s.

Es wurde auch untersucht, wie sich die Richtungsmessung bei sehr niedrigen Ortsdosisleistungen verhält. Auch unterhalb von 0,05 $\mu$Sv/h sind qualitativ hochwertige Richtungsmessungen möglich, die erforderlichen Messzeiten werden dann aber länger und liegen evt. über den mit $t_{90} = T(\text{Nuklid}, \sigma) \cdot \left( \frac{\dot{H}^*(10)}{\mu Sv/h} \right)^{-1}$ berechneten Werten. Mit dem zur Verfügung stehenden Vorrichtungsmodell war es ohne Schwierigkeiten möglich, auch bei wenigen nSv/h die Richtung einer Quelle zuverlässig mit $\pm 5°$ Genauigkeit zu messen. Die Messzeiten liegen dann im Bereich von einigen Minuten. Beispielsweise erreichte die Richtungsmessung für eine Co-60 Quelle mit 3 nSv/h Dosisleistung nach 140 s eine Genauigkeit von $\pm 10°$ und nach 220 s eine Genauigkeit von $\pm 5°$. Eine Cs-137 Quelle mit 4 nSv/h Dosisleistung am Messort konnte nach 55 s mit $\pm 10°$ und nach 90 s mit $\pm 5°$ geortet werden. Die Richtungsmessung einer Co-57 Quelle mit 5 nSv/h Dosisleistung hatte nach 22 s eine Genauigkeit von $\pm 10°$ und nach 35 s eine Genauigkeit von $\pm 5°$.

Bei Vorliegen von quasistationären Messbedingungen kann die Richtungsmessung getaktet ausgeführt werden. Es ist dann z.B. möglich, die Bewegung von Strahlungsquellen zu verfolgen. Die Dosisleistung $\dot{H}^*(10)$ ist wiederum eine geeignete Größe, um die Taktfrequenz zu regeln. Wenn beispielsweise die Bewegung einer Punktquelle verfolgt werden soll, kann die Taktfrequenz auf die $t_{90}$ Zeit eingestellt werden.

Für ein Experiment bei quasistationären Messbedingungen wurde das Vorrichtungsmodell von Abb. 4a auf einen Drehteller montiert. Die Taktfrequenz des Messprogramms wurde auf die $t_{90}$ Zeit eingestellt. Bei einer Drehgeschwindigkeit von 0,2 Umdrehungen pro Minute konnte die aktuelle Position einer 10 $\mu$Ci Co-60 Quelle in 1 Meter Abstand gut verfolgt werden.

## Patentansprüche

1. Verfahren zur mehrdimensionalen Richtungsmessung von Gammastrahlung im Fernfeld mittels einer Gruppe von mehreren energiediskriminierenden, miteinander synchronisierten Detektoren (1, 2) zur Erfassung von Strahlung, wobei

   das Verfahren uni- und bidirektionale Compton Streuprozesse nutzt und Lookup-Tabellen $LUT^{SK}$, einen definierten Funktionswert f(E1,E2), eine Liste von definierten Detektorpaaren mit einer Identifikationsnummer $i$ für definierte Detektorpaare und eine oder mehrere Häufigkeitsverteilungen $\underline{Y}$ zur Erfassung der Messwerte verwendet und dabei die folgenden Schritte umfasst:

   **a) Einrichten** des Detektorsystems für eine Messung, wobei die folgende Abfolge von Schritten ausgeführt wird:

   - **Erstellen** einer Liste mit definierten Detektorpaaren, wobei die definierten Detektorpaare alle Paare umfassen, welche kombinatorisch aus der Menge der Detektoren gebildet werden und jedes Paar mindestens einen Detektor aus einem Material mit einer Ordnungszahl von $Z_{eff} > 30$ enthält, und diese mit einer ID-Nummer $i$ versieht;
   - **Verschalten** aller Detektoren in einer Koinzidenzschaltung derart, dass Koinzidenzereignisse in allen definierten Detektorpaaren $i$= 1, ...,$I$ erfasst werden;
   - **Kennzeichnen** der beiden Detektoren eines jeden definierten Detektorpaares $i$ mit den Nummern 1 bzw. 2, wobei der Detektor mit der niedrigeren Ordnungszahl die Nummer 1 und derjenige mit der höheren Ordnungszahl die Nummer 2 erhält, bestehen beide Detektoren aus dem gleichen Material, wird die Kennzeichnung als 1 bzw. 2 willkürlich getroffen;
   - **Definieren** einer Funktion f(E1,E2) = (E2-E1) / (E1+E2) welche aus zwei Energiewerten E1 und E2 berechnet wird;

   **b) Akquirieren von Messwerten** von Koinzidenzereignissen, wenn in je zwei Detektoren von allen definierten Detektorpaaren $i$ gleichzeitig Wechselwirkungen stattfinden, wobei die Messwerte einer Strahlungsverteilung im Fernfeld entstammen und die Messwerte die in den Detektoren gemessenen Wechselwirkungsenergien E1 und E2 der Strahlung sind;

**c) Assoziieren** von Koinzidenzereignissen mit einer Identifikationsnummer $i$;

**d) Berechnen** des Funktionswertes f(E1,E2) aus zwei Energiewerten E1, E2 pro Koinzidenzereignis mit der in Schritt a) definierten Funktion f(E1,E2);

**e) Erfassen** der Koinzidenzereignisse entsprechend ihrer Identifikationsnummer $i$ und ihrer Funktionswerte f(E1,E2) in einer oder mehreren Häufigkeitsverteilungen $\underline{Y}$, wobei für jedes Radionuklid eine separate Häufigkeitsverteilung $\underline{Y}$ vorliegt,

**f) Berechnen** einer oder mehrerer Richtungsverteilungen $\underline{X}$ aus den Häufigkeitsverteilungen $\underline{Y}$ mit einem statistischen Bildrekonstruktionsverfahren der Emissionstomographie unter Nutzung von Lookup-Tabellen $LUT^{SK}$, wobei für jedes Radionuklid eine separate Richtungsverteilung $\underline{X}$ vorliegt.

2. Verfahren gemäß Anspruch 1, **dadurch gekennzeichnet, dass** die Strahlungsquellen eine diskrete und/oder kontinuierliche Verteilung von Strahlung emittieren;

und/oder dass die Strahlungsquellen neben Gammastrahlung auch Partikelstrahlung aus einer Partikelgruppe von Elektronen, Positronen, Protonen, Ionen und/oder Neutronen emittieren;
und/oder dass die Strahlung aus dem radioaktiven Zerfall eines oder mehrerer Radionuklide stammt;
und/oder dass die Strahlung die prompte Gammastrahlung ist, welche bei der Absorption von Protonen- oder Ionenstrahlung in Targetmaterialien entsteht;
und/oder dass die Strahlung von niedriger Intensität ist.

3. Verfahren gemäß Anspruch 1 oder 2, wobei das Verfahren in Schritt a) zusätzlich in Teilen oder in Gänze die folgende Abfolge von Schritten umfasst:

- **Kalibrieren** der Signale von allen Detektoren als absorbierte Strahlungsenergie E;
- **Festlegen** eines geeigneten Koordinatensystems;
- **Erfassen** der Richtungen der definierten Detektorpaare, wobei in der 2-dimensionalen Richtungsmessung die Richtung von Detektorpaar $i$ mit dem Azimutwinkel $\varphi_i$ erfasst wird und wobei in der 3-dimensionalen Richtungsmessung die Richtung von Detektorpaar $i$ mit dem Azimutwinkel $\varphi_i$ und dem Höhenwinkel $\beta_i$ erfasst wird;
- **Einteilen** des Messbereiches für den Funktionswert f(E1,E2) in eine Anzahl $J$ äquidistante Messwertkanäle $j = 1, ...,J$;
- **Anlegen** eines oder mehrerer 2-dimensionaler Arrays mit $I \cdot J$ Feldern als Datenstrukturen zur Speicherung der Häufigkeitsverteilungen $\underline{Y}$, in welchen Koinzidenzereignisse entsprechend ihrer ID-Nummer $i$ und ihres Messwertkanals $j$ registriert werden; für jedes Radionuklid wird ein separates 2-dimensionales Array $\underline{Y}$ angelegt;
- **Einteilen** der Detektorpaare $i$ in Symmetrieklassen $SK$, wobei diejenigen Detektorpaare $i$, die bei Drehung oder Verschiebung auf andere baugleiche Detektorpaare abgebildet werden, zu jeweils einer Symmetrieklasse $SK(i)$ zusammengefasst werden;
- **Anlegen** einer oder mehrerer Lookup-Tabellen $LUT^{SK}$ für jede Symmetrieklasse $SK$ und jedes Radionuklid, wobei diese einen Winkelbereich $\vartheta$ von 0° bis 180° umspannen und in äquidistante Winkelschritte eingeteilt werden;
- **Erstellen der** Lookup-Tabellen $LUT^{SK}$ durch Messungen mit dem Detektorsystem oder durch Monte Carlo Simulationen oder mittels eines theoretischen Modells;
- **Übergeben** sämtlicher Lookup-Tabellen $LUT^{SK}$ für alle Symmetrieklassen und alle Radionuklide an einen Algorithmus der Bildrekonstruktion, welcher die Messdaten verarbeitet und die Richtungsverteilungen $\underline{X}$ berechnet.

4. Verfahren gemäß Anspruch 3, **dadurch gekennzeichnet, dass** die Lookup-Tabellen $LUT^{SK}$ durch Messungen mit dem Detektorsystem erzeugt werden, indem die folgende Abfolge von Schritten ausgeführt wird:

- **Erstellen** einer Vorschrift zur Erzeugung und Validierung der Lookup-Tabellen $LUT^{SK}$ aus Referenzmessungen, welche die Referenzquellen auswählt, den Nuklidtyp berücksichtigt, sowie die Messbedingungen definiert, unter denen die Messungen durchzuführen sind;
- **Ausführen** von Referenzmessungen für alle in der Vorschrift zuvor definierten Schritte;
- **Erstellen** und **Validieren** der Lookup-Tabellen $LUT^{SK}$ gemäß der zuvor definierten Vorschrift zur Auswertung der Messdaten aus den Referenzmessungen;
- **Erfassen** des natürlichen Strahlungshintergrundes $b^{SK(i)}$ für alle Symmetrieklassen $SK$;
- **Exkludieren** des natürlichen Strahlungshintergrundes $b^{SK}$ aus den Lookup-Tabellen $LUT^{SK}$.

5.

5. Verfahren gemäß einem der Ansprüche 1 - 4, **dadurch gekennzeichnet, dass** in Schritt e) eine Selektionsbedingung bezüglich der Energiesumme E1+E2 der in beiden Detektoren eines Paares detektierten Energien angewendet wird;

   und/oder dass für jedes Radionuklid eine separate Häufigkeitsverteilung **_Y_** angelegt wird;
   und/oder dass für jedes Radionuklid eine separate Richtungsverteilung **_X_** berechnet wird.

6. Verfahren gemäß einem der Ansprüche 1 - 5, **dadurch gekennzeichnet, dass** in Schritt e) bei Radionukliden mit mehreren Gammaenergien mehrere Selektionsbedingungen bezüglich der Energiesumme E1+E2 angewendet werden;
   und/oder dass für solche Radionuklide mit mehreren Gammaenergien eine oder mehrere Häufigkeitsverteilungen **_Y_** angelegt werden.

7. Verfahren gemäß einem der Ansprüche 1 - 6, **dadurch gekennzeichnet, dass** in Schritt f) das statistische Bildrekonstruktionsverfahren ein Teil von oder in Gänze das Maximum Likelihood Expectation Maximization (MLEM) Verfahren, das Ordered Subset Expectation Maximization (OSEM) Verfahren, das List Mode - Maximum Likelihood Expectation Maximization (LM-MLEM) Verfahren und/oder das List Mode - Ordered Subset Expectation Maximization (LM-OSEM) Verfahren ist.

8. Verfahren gemäß einem der Ansprüche 1 - 7, **dadurch gekennzeichnet, dass** in Schritt f) für jedes detektierte Radionuklid eine 2-dimensionale Richtungsverteilung $X_k = X(\omega_k)$ gemäß

$$X_k^{[n+1]} = X_k^{[n]} \frac{K}{\sum_{i,j} Y_{ij}} \sum_{i,j} \frac{LUT_j^{SK(i)} \left[\vartheta(\omega_k, \varphi_i)\right] Y_{ij}}{\sum_{k'=1}^{K} LUT_j^{SK(i)} \left[\vartheta(\omega_{k'}, \varphi_i)\right] X_{k'}^{[n]}}$$

aus einer Anfangsverteilung

$$\forall\, k = 1, \dots, K \qquad X_k^{[0]} = 1$$

unter Nutzung von Lookup-Tabellen $LUT^{SK}$ und einer Winkelabstandsfunktion gemäß

$$\vartheta(\omega_k, \varphi_i) = \arccos\left(\cos(\omega_k - \varphi_i)\right)$$

berechnet wird, wobei K eine vom Nutzer definierte Anzahl an Pixeln für den Azimutwinkel $\omega_k$ ist.

9. Verfahren gemäß einem der Ansprüche 1 - 8, **dadurch gekennzeichnet, dass** in Schritt f) für jedes detektierte Radionuklid eine 3-dimensionale Richtungsverteilung $X_{kl} = X(\omega_k, h_l)$ gemäß

$$X_{kl}^{[n+1]} = X_{kl}^{[n]} \frac{KL}{\sum_{i,j} Y_{ij}} \sum_{i,j} \frac{LUT_j^{SK(i)} \left[\vartheta(\omega_k, h_l, \varphi_i, \beta_i)\right] Y_{ij}}{\sum_{k'=1}^{K} \sum_{l'=1}^{L} LUT_j^{SK(i)} \left[\vartheta(\omega_{k'}, h_{l'}, \varphi_i, \beta_i)\right] X_{k'l'}^{[n]}}$$

aus einer Anfangsverteilung

$$\forall\, k = 1, \dots, K, l = 1, \dots, L \qquad X_{kl}^{[0]} = 1$$

unter Nutzung von Lookup-Tabellen $LUT^{SK}$ und einer Winkelabstandsfunktion gemäß

$$\vartheta(\omega_k, h_l, \varphi_i, \beta_i) = \arccos\left(\cos\beta_i \cos h_l \cos(\omega_k - \varphi_i) + \sin\beta_i \sin h_l\right)$$

berechnet wird, wobei K und L vom Nutzer definierte Anzahlen an Pixeln für den Azimutwinkel $\omega_k$ und den Höhenwinkel $h_l$ sind.

**10.** Vorrichtung zur Ausführung des Verfahrens zur mehrdimensionalen Richtungsmessung von Gammastrahlung im Fernfeld gemäß einem der Ansprüche 1 - 9 umfassend:

- eine Gruppe von mehreren synchronisierten Detektoren (1, 2) zur Erfassung von Strahlung, wobei mindestens ein Detektormaterial eine Ordnungszahl von $Z_{eff} > 30$ aufweist und alle Detektoren die Energien E messen, welche in Wechselwirkungen der Strahlung mit den Detektormaterialien auftreten;
- eine Systemelektronik, welche Koinzidenzereignisse registriert, wenn in je zwei Detektoren aus einer Liste von definierten Detektorpaaren *i* gleichzeitig Wechselwirkungen stattfinden, wobei die Liste von definierten Detektorpaaren alle Paare umfasst, die kombinatorisch aus der Menge aller Detektoren gebildet werden, und die definierten Detektorpaare mindestens einen Detektor aus einem Material mit einer Ordnungszahl von $Z_{eff} >$ 30 enthalten;
- ein Datenakquisitionssystem, das für beide an einem Koinzidenzereignis beteiligte Detektoren eine Rangordnung festlegt, die einen ersten und einen zweiten Detektor definiert, und die in Koinzidenzereignissen gemessenen Energien (E1,E2) entsprechend ihrer Kennzeichnung 1,2 ordnet und in einer chronologischen Liste mit den Attributen { *i*, E1, E2 } und der Detektionszeit *t* speichert; wobei in jedem Detektorpaar *i* der Detektor mit der niedrigeren Ordnungszahl die Nummer 1 und derjenige mit der höheren Ordnungszahl die Nummer 2 erhält; sollten beide Detektoren eines Paares die gleiche Ordnungszahl haben, wird die Kennzeichnung in 1 bzw. 2 willkürlich getroffen; und
- eine Analyseeinheit, die für jedes Koinzidenzereignis aus den vom Datenakquisitionssystem gespeicherten Energien (E1,E2) einen Funktionswert f(E1,E2) = (E2-E1) / (E1+E2) berechnet und die Koinzidenzereignisse entsprechend ihrer Detektorpaarnummer *i* und ihrer Funktionswerte f(E1,E2) in einer oder mehreren Häufigkeitsverteilungen $\underline{Y}$ erfasst und mit einem statistischen Bildrekonstruktionsverfahren der Emissionstomographie unter Nutzung von Lookup-Tabellen $LUT^{SK}$ aus jeder Häufigkeitsverteilung $\underline{Y}$ je eine Richtungsverteilung $\underline{X}$ des Strahlungsfeldes berechnet, wobei für jedes Radionuklid je eine Häufigkeitsverteilung $\underline{Y}$ und je eine Richtungsverteilung $\underline{X}$ vorliegen.

**11.** Vorrichtung nach Anspruch 10, **dadurch gekennzeichnet, dass** die Vorrichtung teilweise oder komplett als eine Compton Kamera, ein Compton Teleskop, eine Single Plane Compton Kamera, eine Neutronenkamera und/oder eine duale Gamma/Neutronenkamera ausgebildet ist;

und/oder wobei die Gesamtheit aller Detektoren in einem Ring angeordnet sind und/oder wobei im Inneren des Rings kein, ein oder mehrere Zentraldetektoren vorliegen;
und/oder der Ring bevorzugt vier oder fünf und besonders bevorzugt sechs Plastik-Szintillationsdetektoren enthält und/oder sich im Inneren des Rings bevorzugt ein oder zwei Szintillationsdetektoren aus NaI, CsI, $CeBr_3$ und/oder $LaBr_3$ befinden;
und/oder die Szintillationsdetektoren bevorzugt in den Größen 1"×1", 1,5"×1,5", 2"×2" und/oder 3"×3" ausgebildet sind.

**12.** Vorrichtung nach Anspruch 10 oder 11, **dadurch gekennzeichnet, dass** als Detektor ein Szintillationsdetektor verwendet wird und/oder der Szintillator als monolithischer Block oder als pixeliertes Szintilatormodul ausgebildet ist und/oder welcher aus reinen oder dotierten Materialien der Gruppe von PVT, Anthracen, Stilben, p-Terphenyl, $CaF_2$, $BaF_2$, NaI, $CeBr_3$, $LaBr_3$, $LaCl_3$, $La(Br_xCl_{1-x})_3$, CsI, $SrI_2$, CLYC, CLBC, CLCB, CLLB, BGO, LSO, LYSO, GAGG, YAP und/oder YAG gebildet ist;
und/oder dass als Detektor ein Halbleiterdetektor verwendet wird und/oder der Halbleiter als segmentierter oder unsegmentierter Halbleiter ausgebildet ist und/oder welcher eine planare oder koaxiale Geometrie aufweist und/oder welcher aus Materialien der Gruppe von Ge, GaAs, CdTe und/oder CdZnTe gebildet ist.

**13.** Vorrichtung nach einem der Ansprüche 10 - 12, **dadurch gekennzeichnet, dass** mind. zwei Detektoren verwendet werden;
und/oder dass alle verwendeten Detektoren im Wesentlichen baugleich sind oder dass mindestens zwei der verwendeten Detektoren voneinander verschieden sind.

**14.** Vorrichtung nach einem der Ansprüche 10 - 13, **dadurch gekennzeichnet, dass** eine Detektor- und Systemelektronik verwendet wird, die analoge und/oder digitale Elektronikkomponenten benutzt;

und/oder dass die analogen Elektronikkomponenten eine Kombination verschiedener Module umfassen, zu denen eine Hochspannungsversorgung, ein Vorverstärker, ein Verstärker, ein Pulsformer, ein Ladungsintegrator, ein Pulshöhenanalysator, ein Multikanalanalysator (MCA) und/oder eine Koinzidenzschaltung zählen;

und/oder dass die digitalen Elektronikkomponenten eine Kombination verschiedener Hard- und Softwarekomponenten umfassen, zu denen eine Hochspannungsversorgung, ein A/D-Wandler pro Detektor, ein Field Programmable Gate Array (FPGA), ein Speichermedium, ein digitaler Signalprozessor und/oder eine Auswertesoftware zählen.

**Claims**

1. A method for the multidimensional directional measurement of gamma radiation in the far field by means of a group of several energy-discriminating, synchronized detectors (1, 2) for detecting radiation, wherein the method uses unidirectional and bi-directional Compton scattering processes and lookup tables $LUT^{SK}$, a defined functional value f(E1, E2), a list of defined detector pairs with an identification number $i$ for defined detector pairs and one or more frequency distributions $\underline{Y}$ for detecting the measurement values, and comprising the following steps:

   **a) setting up** the detector system for a measurement, wherein the following sequence of steps is performed:

      - **creating** a list of defined detector pairs, wherein the defined detector pairs comprise all pairs formed combinatorially from the set of detectors, each pair contains at least one detector made from a material having an atomic number of $Z_{eff}$ > 30 and identifying it with an ID number $i$;
      - **interconnecting** all detectors in a coincidence circuit such that coincidence events are detected in all defined detector pairs $i$=1,..., $I$;
      - **identifying** both detectors of each defined detector pair $i$ with the numbers 1 and 2, respectively, wherein the detector with the lower atomic number receives the number 1 and the detector with the higher atomic number receives the number 2, both detectors are made of the same material, the identification as 1 and 2 is made arbitrarily;
      - **defining** a function f(E1, E2) = (E2-E1)/(E1+E2) which is calculated from two energy values E1 and E2;

   **b) acquiring measured values** of coincidence events if interactions take place simultaneously in two respective detectors of all defined detector pairs $i$, wherein the measured values originate from a radiation distribution in the far field and the measured values are the interaction energies E1 and E2 of the radiation measured in the detectors;
   **c) associating** coincidence events with an identification number $i$;
   **d) calculating** the functional value f(E1, E2) from two energy values E1, E2 per coincidence event by means of the function f(E1, E2) defined in step a);
   **e) detecting** the coincidence events according to their identification number $i$ and their functional values f(E1, E2) in one or more frequency distributions $\underline{Y}$, wherein a separate frequency distribution $\underline{Y}$ is provided for each radionuclide,
   **f) calculating** one or more directional distributions $\underline{X}$ from the frequency distributions $\underline{Y}$ by means of a statistical image reconstruction method of emission tomography using lookup tables $LUT^{SK}$, wherein a separate directional distribution $\underline{X}$ is provided for each radionuclide.

2. The method according to claim 1, **characterized in that** the radiation sources emit a discrete and/or continuous distribution of radiation;

   and/or **in that** the radiation sources emit, in addition to gamma radiation, particle radiation from a particle group of electrons, positrons, protons, ions and/or neutrons;
   and/or **in that** the radiation originates from the radioactive decay of one or more radionuclides;
   and/or **in that** the radiation is the prompt gamma radiation produced during the absorption of proton or ion radiation in target materials;
   and/or **in that** the radiation is of low intensity.

3. The method according to claim 1 or 2, wherein the method in step a) additionally comprises, in part or in whole, the following sequence of steps:

   - **calibrating** the signals from all detectors as absorbed radiation energy E;
   - **determining** a suitable coordinate system;
   - **detecting** the directions of the defined detector pairs, wherein in the 2-dimensional directional measurement the direction of detector pair $i$ is detected with the azimuth angle $\varphi_i$ and wherein in the 3-dimensional directional measurement the direction of detector pair $i$ is detected with the azimuth angle $\varphi_i$ and the height angle $\beta_i$;

- **dividing** the measurement range for the functional value f(E1, E2) into a number $J$ of equidistant measured value channels $j = 1,..., J$;

- **creating** one or more 2-dimensional arrays with $I \cdot J$ fields as data structures for storing the frequency distributions **$\underline{Y}$**, in which coincidence events are recorded according to their ID number $i$ and their measured value channel $j$; for each radionuclide, a separate 2-dimensional array **$\underline{Y}$**, is created;

- **dividing** the detector pairs $i$ into symmetry classes $SK$, wherein the detector pairs $i$ which are imaged on other identical detector pairs in case of rotation or displacement are combined to form a respective symmetry class $SK$ ($i$);

- **creating** one or more lookup tables $LUT^{SK}$ for each symmetry class $SK$ and each radionuclide, wherein they cover an angular range $\vartheta$ of 0° to 180° and are divided into equidistant angular steps;

- **creating the** lookup tables $LUT^{SK}$ by measurements with the detector system or by Monte Carlo simulations or by means of a theoretical model;

- **transferring** all lookup tables $LUT^{SK}$ for all symmetry classes and all radionuclides to an image reconstruction algorithm that processes the measurement data and calculates the directional distributions **$\underline{X}$**.

4. The method according to claim 3, **characterized in that** the lookup tables $LUT^{SK}$ are generated by measurements with the detector system by performing the following sequence of steps:

- **creating** a specification for the generation and validation of the lookup tables $LUT^{SK}$ from reference measurements, which selects the reference sources, takes into account the nuclide type, and defines the measurement conditions under which the measurements are to be carried out;

- **performing** reference measurements for all steps previously defined in the specification;

- **creating** and **validating** the lookup tables $LUT^{SK}$ according to the previously defined specification for evaluating the measurement data from the reference measurements;

- **detecting** the natural radiation background $b^{SK(i)}$ for all symmetry classes $SK$;

- **excluding** the natural radiation background $b^{SK}$ from the lookup tables $LUT^{SK}$.

5. The method according to any one of claims 1-4, **characterized in that** in step e) a selection condition is applied for the energy sum E1+E2 of the energies detected in both detectors of a pair;

and/or **in that** for each radionuclide a separate frequency distribution **$\underline{Y}$** is created;
and/or **in that** for each radionuclide a separate directional distribution **$\underline{X}$** is calculated.

6. The method according to any one of claims 1-5, **characterized in that**, in step e), in the case of radionuclides having multiple gamma energies, multiple selection conditions are applied with respect to energy sum E1+E2;
and/or **in that** for such radionuclides with multiple gamma energies one or more frequency distributions **$\underline{Y}$** are created.

7. The method according to any one of claims 1-6, **characterized in that** in step f) the statistical image reconstruction method is part or all of the Maximum Likelihood Expectation Maximization (MLEM) method, the Ordered Subset Expectation Maximization (OSEM) method, the List Mode - Maximum Likelihood Expectation Maximization (LM-MLEM) method and/or the List Mode - Ordered Subset Expectation Maximization (LM-OSEM) method.

8. The method according to any one of claims 1-7, **characterized in that** in step f) for each detected radionuclide a 2-dimensional directional distribution $X_k = X(\omega_k)$ is calculated according to

$$X_k^{[n+1]} = X_k^{[n]} \frac{K}{\sum_{i,j} Y_{ij}} \sum_{i,j} \frac{LUT_j^{SK(i)} [\vartheta(\omega_k, \varphi_i)] \; Y_{ij}}{\sum_{k'=1}^{K} LUT_j^{SK(i)} [\vartheta(\omega_{k'}, \varphi_i)] \; X_{k'}^{[n]}}$$

from an initial distribution

$$\forall \, k = 1, ..., K \qquad X_k^{[0]} = 1$$

using lookup tables $LUT^{SK}$ and an angular distance function in accordance with

$$\vartheta(\omega_k, \varphi_i) = \arccos\left(\cos(\omega_k - \varphi_i)\right)$$

wherein K is a user-defined number of pixels for the azimuth angle $\omega_k$.

9. The method according to any one of claims 1-8, **characterized in that** in step f) for each detected radionuclide a 3-dimensional directional distribution $X_{kl} = X(\omega_k, h_l)$ is calculated in accordance with

$$X_{kl}^{[n+1]} = X_{kl}^{[n]} \frac{KL}{\sum_{i,j} Y_{ij}} \sum_{i,j} \frac{LUT_j^{SK(i)} \left[\vartheta(\omega_k, h_l, \varphi_i, \beta_i)\right] Y_{ij}}{\sum_{k'=1}^{K} \sum_{l'=1}^{L} LUT_j^{SK(i)} \left[\vartheta(\omega_{k'}, h_{l'}, \varphi_i, \beta_i)\right] X_{k'l'}^{[n]}}$$

from an initial distribution

$$\forall\, k = 1, \dots, K, l = 1, \dots, L \qquad X_{kl}^{[0]} = 1$$

using lookup tables $LUT^{SK}$ and an angular distance function in accordance with

$$\vartheta(\omega_k, h_l, \varphi_i, \beta_i) = \arccos\left(\cos\beta_i \cos h_l \cos(\omega_k - \varphi_i) + \sin\beta_i \sin h_l\right)$$

wherein K and L are user-defined numbers of pixels for the azimuth angle $\omega_k$ and the height angle $h_l$.

10. A device for carrying out the method of multidimensional directional measurement of gamma radiation in the far field according to any one of claims 1-9 comprising:

- a group of a plurality of synchronized detectors (1, 2) for detecting radiation, wherein at least one detector material has an atomic number of $Z_{eff} > 30$ and all detectors measure the energies E which occur in interactions of the radiation with the detector materials;
- a system electronics which records coincidence events when interactions take place simultaneously in two detectors each from a list of defined detector pairs $i$, wherein the list of defined detector pairs comprises all pairs which are formed combinatorially from the set of all detectors, and the defined detector pairs comprise at least one detector made of a material having an atomic number of $Z_{eff} > 30$;
- a data acquisition system which determines a ranking for both detectors involved in a coincidence event, which ranking defines a first and a second detector, and orders the energies (E1, E2) measured in coincidence events according to their identification 1, 2 and stores them in a chronological list with the attributes {$i$, E1, E2} and the detection time $t$; wherein in each detector pair $i$ the detector with the lower order number is given the number 1 and the detector with the higher order number is given the number 2; if both detectors of a pair have the same order number, the identification as 1 and 2 is arbitrarily taken; and
- an analysis unit which calculates a function value f(E1, E2) = (E2-E1)/(E1+E2) for each coincidence event from the energies (E1, E2) stored by the data acquisition system and detects the coincidence events according to their detector pair number $i$ and their functional values f(E1, E2) in one or more frequency distributions $\underline{Y}$ and using a statistical image reconstruction method of emission tomography using lookup tables $LUT^{SK}$ calculates from each frequency distribution $\underline{Y}$ a respective directional distribution $\underline{X}$ of the radiation field, wherein a respective frequency distribution $\underline{Y}$ is provided for each radionuclide and for each directional distribution $\underline{X}$.

11. The device according to claim 10, **characterized in that** the device is partially or completely formed as a Compton camera, a Compton telescope, a Single Plane Compton camera, a neutron camera and/or a dual gamma/neutron camera;

and/or wherein all the detectors are arranged in a ring and/or wherein inside the ring there are no central detectors, there is one central detector or there are multiple central detectors; and/or the ring preferably contains four or five and particularly preferably six plastic scintillation detectors and/or one or two scintillation detectors of Nal, Csl, CeBr$_3$ and/or LaBr$_3$ are preferably located inside the ring;
and/or the scintillation detectors are preferably designed in the sizes 1"×1", 1.5"×1.5", 2"×2" and/or 3"×3".

12. The device according to claim 10 or 11, **characterized in that** a scintillation detector is used as detector and/or the scintillator is formed as monolithic block or as a pixelated scintillator module and/or is formed from pure or doped materials of the group of PVT, anthracene, stilbene, p-terphenyl, $CaF_2$, $BaF_2$, Nal, $CeBr_3$, $LaBr_3$, $LaCl_3$, $La(Br_xCl_{1-x})_3$, CsI, $SrI_2$, CLYC, CLBC, CLCB, CLLB, BGO, LSO, LYSO, GAGG, YAP and/or YAG; and/or **in that** a semiconductor detector is used as the detector and/or the semiconductor is designed as a segmented or unsegmented semiconductor and/or has a planar or coaxial geometry and/or is formed from materials of the group of Ge, GaAs, CdTe and/or CdZnTe.

13. The device according to any one of claims 10-12, **characterized in that** at least two detectors are used; and/or **in that** all the detectors used are substantially identical in construction or that at least two of the detectors used are different from one another.

14. The device according to any one of claims 10-13, **characterized in that** detector and system electronics used use analog and/or digital electronic components;

and/or **in that** the analog electronic components comprise a combination of different modules including a high voltage supply, a preamplifier, an amplifier, a pulse shaper, a charge integrator, a pulse height analyzer, a multichannel analyzer (MCA) and/or a coincidence circuit;
and/or **in that** the digital electronic components comprise a combination of different hardware and software components, which comprise a high-voltage supply, an A/D converter per detector, a field programmable gate array (FPGA), a storage medium, a digital signal processor and/or an evaluation software.

## Revendications

1. Procédé de mesure directionnelle multidimensionnelle du rayonnement gamma dans le champ lointain au moyen d'un groupe de plusieurs détecteurs (1, 2) synchronisés entre eux et discriminant l'énergie, pour détecter un rayonnement, dans lequel le procédé utilise des processus de diffusion Compton unidirectionnels et bidirectionnels et des tables de consultation $LUT^{SK}$, une valeur de fonction définie f(E1, E2), une liste de paires de détecteurs définies avec un numéro d'identification $i$ pour des paires de détecteurs définies et une ou plusieurs répartitions de fréquence $\underline{Y}$ pour enregistrer des mesures, comprenant les étapes suivantes :

a) **la configuration** du système de détection pour une mesure, dans lequel la séquence d'étapes suivante est exécutée :

- **l'établissement** d'une liste de paires de détecteurs définies, dans lequel les paires de détecteurs définies comprennent toutes les paires formées de manière combinatoire à partir de l'ensemble des détecteurs et chaque paire contient au moins un détecteur constitué d'un matériau avec un numéro atomique de $Z_{eff} > 30$ et leur attribue un numéro d'identification $i$ ;
- **la commutation** de tous les détecteurs dans un circuit de coïncidence de telle sorte que les événements de coïncidence soient détectés dans toutes les paires de détecteurs définies $i = 1,..., I$ ;
- **l'identification** des deux détecteurs de chaque paire de détecteurs définie $i$ par les numéros 1 et 2, dans lequel le détecteur ayant le numéro atomique le plus faible reçoit le numéro 1 et celui ayant le numéro atomique le plus élevé reçoit le numéro 2, si les deux détecteurs sont constitués du même matériau, l'identification par les numéros 1 et 2 est appliquée de manière arbitraire ;
- **la définition** d'une fonction f(E1, E2) = (E2-E1) / (E1+E2) calculée à partir de deux valeurs d'énergie E1 et E2 ;

b) **l'acquisition de mesures** d'événements de coïncidence lorsque des interactions se produisent simultanément dans deux détecteurs de chacune des paires de détecteurs $i$ définies, dans lequel les mesures proviennent d'une distribution de rayonnement dans le champ lointain et les mesures sont les énergies d'interaction E1 et E2 du rayonnement mesurées dans les détecteurs ;
c) **l'association** d'événements de coïncidence à un numéro d'identification $i$ ;
d) **le calcul** de la valeur de fonction f(E1, E2) à partir de deux valeurs d'énergie E1, E2 par événement de coïncidence avec la fonction f(E1, E2) définie à l'étape a) ;
e) **l'enregistrement** des événements de coïncidence selon leur numéro d'identification $i$ et leurs valeurs de fonction f(E1, E2) dans une ou plusieurs distributions de fréquence $\underline{Y}$, dans lequel chaque radionucléide présente une distribution de fréquence distincte $\underline{Y}$,

**f) le calcul** d'une ou de plusieurs distributions directionnelles $X$ des distributions de fréquence $Y$ à l'aide d'un procédé statistique de reconstruction d'image de la tomographie d'émission utilisant des tables de consultation $LUT^{SK}$, dans lequel chaque radionucléide présente une distribution directionnelle distincte $X$.

2. Procédé selon la revendication 1, **caractérisé en ce que** les sources de rayonnement émettent une distribution distincte et/ou continue de rayonnement ;

et/ou que les sources de rayonnement émettent, en plus du rayonnement gamma, des rayonnements particulaires provenant d'un groupe de particules d'électrons, de positrons, de protons, d'ions et/ou de neutrons ;
et/ou que les rayonnements proviennent de la décomposition radioactive d'un ou de plusieurs radionucléides ;
et/ou que le rayonnement est le rayonnement gamma instantané résultant de l'absorption du rayonnement protonique ou ionique dans les matériaux cibles ;
et/ou que le rayonnement est de faible intensité.

3. Procédé selon la revendication 1 ou 2, dans lequel le procédé de l'étape a) comprend également, en partie ou en totalité, la séquence d'étapes suivante :

- **l'étalonnage** des signaux de tous les détecteurs en tant qu'énergie rayonnante absorbée E ;
- **la définition** d'un système de coordonnées approprié ;
- **la détection** des directions des paires de détecteurs définies, dans lequel, dans la mesure directionnelle à deux dimensions, la direction de la paire de détecteurs $i$ est détectée avec l'angle azimutal $\varphi_i$ et dans lequel, dans la mesure directionnelle à trois dimensions, la direction de la paire de détecteurs $i$ est détectée avec l'angle azimutal $\varphi_i$ et l'angle d' élévation $\beta_i$ ;
- **la division** de l'étendue de mesure pour la valeur de fonction f(E1, E2) en un nombre J de canaux de mesure équidistants $j$ = 1,..., $J$ ;
- **la création** d'un ou plusieurs tableaux à deux dimensions comportant I · J champs comme structures de données pour stocker les distributions de fréquence $Y$ dans lesquelles les événements de coïncidence sont enregistrés en fonction de leur numéro d'identification $i$ et de leur canal de mesure $j$ $Y$ ; un tableau à deux dimensions distinct étant créé pour chaque radionucléide ;
- **la division** des paires de détecteurs $i$ en classes de symétrie $SK$, dans lequel les paires de détecteurs $i$, qui sont mappées sur d'autres paires de détecteurs de même construction lors de la rotation ou du déplacement, sont chacune regroupées en une classe de symétrie $SK(i)$ ;
- **la création** d'une ou de plusieurs tables de consultation $LUT^{SK}$ pour chaque classe de symétrie SK et chaque radionucléide, dans lequel celles-ci couvrent une plage d'angles $\vartheta$ comprise entre 0° et 180° et sont divisées en paliers angulaires équidistants ;
- **la création des** tables de consultation $LUT^{SK}$ par des mesures effectuées avec le système de détection ou par des simulations Monte-Carlo ou par un modèle théorique ;
- **la transmission** de toutes les tables de consultation $LUT^{SK}$ pour toutes les classes de symétrie et tous les radionucléides à un algorithme de reconstruction d'image qui traite les données de mesure et calcule les distributions directionnelles $X$.

4. Procédé selon la revendication 3, **caractérisé en ce que** les tables de consultation $LUT^{SK}$ sont produites par des mesures avec le système de détection en exécutant la séquence d'étapes suivante :

- **la création** d'une procédure de production et de validation des tables de consultation $LUT^{SK}$ à partir de mesures de référence, en sélectionnant les sources de référence, en tenant compte du type de nucléide et en définissant les conditions de mesure dans lesquelles les mesures doivent être effectuées ;
- **la réalisation** des mesures de référence pour toutes les étapes prédéfinies dans la procédure ;
- **la création** et la **validation** des tables de consultation $LUT^{SK}$ conformément à la procédure prédéfinie d'évaluation des données de mesure des mesures de référence ;
- **l'enregistrement** de l'arrière-plan de rayonnement naturel $b^{SK(i)}$ pour toutes les classes de symétrie SK ;
- **l'exclusion** de l'arrière-plan de rayonnement naturel $b^{SK}$ des tables de consultation $LUT^{SK}$.

5. Procédé selon l'une des revendications 1 à 4, **caractérisé en ce que**, à l'étape e), une condition de sélection est appliquée concernant la somme d'énergie E1+E2 des énergies détectées dans les deux détecteurs d'une paire ;

et/ou **en ce qu'**une distribution de fréquence distincte pour chaque radionucléide $Y$ est créée ;
et/ou **en ce qu'**une distribution de direction distincte pour chaque radionucléide $X$ est calculée.

**6.** Procédé selon l'une des revendications 1 à 5, **caractérisé en ce que**, à l'étape e), plusieurs conditions de sélection sont appliquées concernant la somme d'énergie E1+E2 pour des radionucléides ayant plusieurs énergies gamma ; et/ou **en ce qu'**une ou plusieurs distributions de fréquences $\underline{Y}$ soient créées pour de tels radionucléides avec plusieurs énergies gamma.

**7.** Procédé selon l'une des revendications 1 à 6, **caractérisé en ce que**, à l'étape f), le procédé de reconstruction statistique d'image est une partie ou la totalité du procédé MLEM (Maximum Likelihood Expectation Maximization), du procédé OSEM (Ordered Subset Expectation Maximum), du procédé LM-MLEM (List Mode - Maximum Likelihood Expectation Maximization) et/ou du procédé LM-OSEM (List Mode - Ordered Subset Expectation Maximization).

**8.** Procédé selon l'une des revendications 1 à 7, **caractérisé en ce qu'**à l'étape f), pour chaque radionucléide détecté, une distribution directionnelle à deux dimensions $X_k = X(\omega_k)$ est calculée selon

$$X_k^{[n+1]} = X_k^{[n]} \frac{K}{\sum_{i,j} Y_{ij}} \sum_{i,j} \frac{LUT_j^{SK(i)}\left[\vartheta(\omega_k, \varphi_i)\right] Y_{ij}}{\sum_{k'=1}^{K} LUT_j^{SK(i)}\left[\vartheta(\omega_{k'}, \varphi_i)\right] X_{k'}^{[n]}}$$

à partir d'une distribution initiale

$$\forall\, k = 1, \dots, K \qquad X_k^{[0]} = 1$$

en utilisant des tables de consultation $LUT^{SK}$ et une fonction de distance angulaire selon

$$\vartheta(\omega_k, \varphi_i) = \arccos\left(\cos(\omega_k - \varphi_i)\right)$$

dans lequel K est un nombre de pixels défini par l'utilisateur pour l'angle azimutal $\omega_k$.

**9.** Procédé selon l'une des revendications 1 à 8, **caractérisé en ce qu'**à l'étape f), pour chaque radionucléide détecté, une distribution directionnelle à trois dimensions $X_{kl} = X(\omega_k, h_1)$ est calculée selon

$$X_{kl}^{[n+1]} = X_{kl}^{[n]} \frac{KL}{\sum_{i,j} Y_{ij}} \sum_{i,j} \frac{LUT_j^{SK(i)}\left[\vartheta(\omega_k, h_l, \varphi_i, \beta_i)\right] Y_{ij}}{\sum_{k'=1}^{K} \sum_{l'=1}^{L} LUT_j^{SK(i)}\left[\vartheta(\omega_{k'}, h_{l'}, \varphi_i, \beta_i)\right] X_{k'l'}^{[n]}}$$

à partir d'une distribution initiale

$$\forall\, k = 1, \dots, K, l = 1, \dots, L \qquad X_{kl}^{[0]} = 1$$

en utilisant des tables de consultation $LUT^{SK}$ et une fonction de distance angulaire selon

$$\vartheta(\omega_k, h_l, \varphi_i, \beta_i) = \arccos\left(\cos\beta_i \cos h_l \cos(\omega_k - \varphi_i) + \sin\beta_i \sin h_l\right)$$

dans lequel K et L sont des nombres de pixels définis par l'utilisateur pour l'angle azimutal $\omega_k$ et l'angle d'élévation $h_1$.

**10.** Dispositif pour la mise en œuvre du procédé de mesure directionnelle multidimensionnelle du rayonnement gamma dans le champ lointain selon l'une des revendications 1 à 9, comprenant :

- un groupe de plusieurs détecteurs synchronisés (1, 2) pour détecter un rayonnement, dans lequel au moins un matériau détecteur comporte un numéro atomique de $Z_{eff} > 30$ et tous les détecteurs mesurent les énergies E qui se produisent dans les interactions du rayonnement avec les matériaux détecteurs ;
- une électronique de système qui enregistre des événements de coïncidence lorsque des interactions se produisent simultanément dans deux détecteurs d'une liste de paires de détecteurs *i* définies, dans lequel la liste de paires de détecteurs définies comprend toutes les paires formées de manière combinatoire à partir de l'ensemble de tous les détecteurs, et les paires de détecteurs définies comprennent au moins un détecteur d'un matériau ayant un numéro atomique de $Z_{eff} > 30$ ;

- un système d'acquisition de données qui définit, pour les deux détecteurs impliqués dans un événement de coïncidence, un ordre de priorité définissant un premier et un second détecteur et qui classe les énergies mesurées dans les événements de coïncidence (E1, E2) selon leur identification 1, 2 et les stocke dans une liste chronologique avec les attributs {i, E1, E2} et le temps de détection t ; dans lequel, dans chaque paire de détecteurs *i*, le détecteur ayant le numéro atomique le plus bas reçoit le numéro 1 et celui ayant le numéro atomique le plus élevé reçoit le numéro 2 ; si les deux détecteurs d'une paire ont le même numéro atomique, l'identification par les numéros 1 et 2 est effectuée de manière arbitraire ; et

- une unité d'analyse qui calcule, pour chaque événement de coïncidence, à partir des énergies (E1, E2) stockées par le système d'acquisition de données, une valeur de fonction f(E1, E2) = (E2-E1) / (E1+E2) et qui détecte les événements de coïncidence selon leur numéro de paire de détecteurs *i* et leurs valeurs de fonction f(E1, E2) dans une ou plusieurs distributions de fréquence $\underline{Y}$ et qui calcule une distribution directionnelle $\underline{X}$ du champ de rayonnement par un procédé statistique de reconstruction d'image de la tomographie d'émission utilisant des tables de consultation $LUT^{SK}$ de chaque distribution de fréquence $\underline{Y}$, dans lequel une distribution de fréquence $\underline{Y}$ et une distribution directionnelle $\underline{X}$ sont présentes pour chaque radionucléide.

11. Dispositif selon la revendication 10, **caractérisé en ce que** le dispositif est réalisé en partie ou en totalité sous la forme d'une caméra Compton, d'un télescope Compton, d'une caméra Compton monoplan, d'une caméra à neutrons et/ou d'une double caméra gamma-neutrons ;

et/ou dans lequel l'ensemble des détecteurs sont disposés dans un cercle et/ou dans lequel il n'y a pas un ou plusieurs détecteurs centraux à l'intérieur du cercle ;
et/ou le cercle contient de préférence quatre ou cinq détecteurs de scintillation en matière plastique et de manière particulièrement préférée six, et/ou il y a de préférence à l'intérieur de l'anneau un ou deux détecteurs de scintillation en NaI, CsI, CeBr$_3$ et/ou LaBr$_3$ ;
et/ou les détecteurs à scintillation sont de préférence de tailles 1×1", 1,5"×1,5", 2"×2" et/ou 3"×3".

12. Dispositif selon la revendication 10 ou 11, **caractérisé en ce qu'**un détecteur de scintillation est utilisé comme détecteur et/ou le scintillateur est réalisé sous forme de bloc monolithique ou de module de scintillateur pixellisé et/ou qui est constitué de matériaux purs ou dopés du groupe constitué de : PVT, anthracène, stilbène, p-terphényle, CaF$_2$, BaF$_2$, NaI, CeBr$_3$, LaBr$_3$, LaCl$_3$, La(Br$_x$Cl$_{1-x}$)$_3$, CsI, SrI$_2$, CLYC, CLBC, CLCB, CLLB, BGO, LSO, LYSO, GAGG, YAP et/ou YAG ;
et/ou **en ce qu'**un détecteur à semi-conducteur est utilisé comme détecteur et/ou que le semi-conducteur est réalisé sous la forme d'un semi-conducteur segmenté ou non segmenté et/ou qui présente une géométrie plane ou coaxiale et/ou qui est formé de matériaux du groupe de Ge, GaAs, CdTe et/ou CdZnTe.

13. Dispositif selon l'une des revendications 10 à 12, **caractérisé en ce qu'**au moins deux détecteurs sont utilisés ; et/ou que tous les détecteurs utilisés sont essentiellement de même construction ou qu'au moins deux des détecteurs utilisés sont différents.

14. Dispositif selon l'une des revendications 10 à 13, **caractérisé en ce qu'**une électronique de détection et de système est utilisée qui utilise des composants électroniques analogiques et/ou numériques ;

et/ou **en ce que** les composants électroniques analogiques comprennent une combinaison de différents modules, y compris une alimentation haute tension, un préamplificateur, un amplificateur, un formateur d'impulsions, un intégrateur de charge, un analyseur de hauteur d'impulsion, un analyseur multicanal (MCA) et/ou un circuit de coïncidence ;
et/ou **en ce que** les composants électroniques numériques comprennent une combinaison de différents composants matériels et logiciels, y compris une alimentation haute tension, un convertisseur A/N par détecteur, un réseau de portes programmables en champ (FPGA), un support de stockage, un processeur de signaux numériques et/ou un logiciel d'évaluation.

Abb. 1

Abb. 2

Abb. 3

(a)                                   (b)

Abb. 4a                               Abb. 4b

Häufigkeitsverteilung Y̲ für die 2-dimensionale Richtungsmessung

Detektorpaare I bis VI
1=Plastik, 2=CeBr$_3$

| A | I 0° | II 60° | III 120° | IV 180° | V 240° | VI 300° |
|---|---|---|---|---|---|---|
| - 1,0 | | | | | | |
| - 0,9 | | 1 | | 1 | | |
| - 0,8 | 1 | | | 1 | | |
| - 0,7 | | | 1 | 2 | | |
| - 0,6 | | | 2 | | 1 | |
| - 0,5 | | | | 1 | | 1 |
| - 0,4 | | | | | | |
| - 0,3 | | | | 2 | | |
| - 0,2 | | 1 | 1 | 2 | | 1 |
| - 0,1 | 1 | | 1 | | | |
| 0,0 | 1 | | | | | |
| + 0,1 | | | | 1 | | 1 |
| + 0,2 | | | 1 | 1 | | |
| + 0,3 | | | 1 | | | |
| + 0,4 | 2 | | 2 | | | |
| + 0,5 | 2 | | 1 | | | |
| + 0,6 | 4 | | | 1 | | |
| + 0,7 | 1 | 2 | | | | |
| + 0,8 | 1 | 3 | | | | |
| + 0,9 | | 1 | | | 1 | 3 |
| + 1,0 | 2 | 5 | 3 | 1 | 3 | |

$$A = \frac{E2 - E1}{E1 + E2}$$

Abb. 5

(a)

(b)

Abb. 6a

Abb. 6b

Häufigkeitsverteilung Y̲ für die 3-dimensionale Richtungsmessung

$$A = \frac{E2 - E1}{E1 + E2}$$

**SK=1**

Detektorpaare I bis VI
1=Plastik, 2=CeBr₃ oben

| A | I | II | III | IV | V | VI |
|---|---|----|-----|----|---|----|
| - 1,0 |   |   |   |   |   |   |
| - 0,9 |   | 1 |   | 1 |   |   |
| - 0,8 | 1 |   |   | 1 | 1 |   |
| - 0,7 |   |   | 1 | 2 |   |   |
| - 0,6 |   |   | 2 |   | 1 |   |
| - 0,5 |   |   |   | 1 |   | 1 |
| - 0,4 | 1 |   |   |   |   |   |
| - 0,3 | 1 |   |   | 2 |   |   |
| - 0,2 |   | 1 | 1 | 2 |   | 1 |
| - 0,1 | 2 |   | 1 |   | 2 | 1 |
| 0,0 |   | 4 |   |   |   |   |
| + 0,1 | 1 |   |   | 1 | 3 | 1 |
| + 0,2 | 1 |   | 1 | 1 |   |   |
| + 0,3 |   | 7 | 1 |   |   |   |
| + 0,4 | 2 | 6 | 2 | 5 |   |   |
| + 0,5 | 2 |   | 1 |   |   |   |
| + 0,6 | 8 |   |   | 1 |   |   |
| + 0,7 | 1 | 2 |   |   |   |   |
| + 0,8 | 1 | 3 | 6 |   |   |   |
| + 0,9 | 2 | 1 | 4 | 8 | 5 | 3 |
| + 1,0 | 2 | 9 | 7 | 1 | 3 | 5 |

**SK=2**

Detektorpaare VII bis XII
1=Plastik, 2=CeBr₃ unten

| A | VII | VIII | IX | X | XI | XII |
|---|-----|------|----|---|----|-----|
| - 1,0 |   |   |   |   |   |   |
| - 0,9 |   | 1 |   | 1 |   |   |
| - 0,8 | 1 |   |   | 1 |   | 1 |
| - 0,7 |   |   | 1 | 2 |   | 1 |
| - 0,6 |   | 1 | 2 |   |   |   |
| - 0,5 | 2 | 3 |   | 1 | 1 |   |
| - 0,4 |   | 1 | 2 |   |   |   |
| - 0,3 |   |   |   | 1 |   |   |
| - 0,2 |   | 1 | 1 |   | 2 |   |
| - 0,1 | 1 |   | 1 |   | 3 |   |
| 0,0 | 1 |   |   | 2 |   | 1 |
| + 0,1 |   |   |   | 1 |   |   |
| + 0,2 |   | 3 | 1 | 1 |   |   |
| + 0,3 |   | 1 |   |   |   |   |
| + 0,4 | 3 |   | 2 |   |   |   |
| + 0,5 | 2 |   | 1 |   |   |   |
| + 0,6 | 1 |   |   | 1 | 5 |   |
| + 0,7 | 1 | 2 |   |   |   | 1 |
| + 0,8 | 2 | 5 | 4 | 7 | 8 | 6 |
| + 0,9 | 3 | 1 | 3 | 4 | 1 | 3 |
| + 1,0 | 2 | 5 | 3 | 1 | 3 | 2 |

**SK=3**

Detektorpaar XIII
1=CeBr₃ oben, 2=CeBr₃ unten

| A | XIII |
|---|------|
| - 1,0 |   |
| - 0,9 |   |
| - 0,8 | 1 |
| - 0,7 |   |
| - 0,6 |   |
| - 0,5 | 3 |
| - 0,4 | 2 |
| - 0,3 |   |
| - 0,2 |   |
| - 0,1 | 1 |
| 0,0 | 1 |
| + 0,1 |   |
| + 0,2 |   |
| + 0,3 | 2 |
| + 0,4 | 4 |
| + 0,5 | 7 |
| + 0,6 | 4 |
| + 0,7 | 3 |
| + 0,8 | 2 |
| + 0,9 | 3 |
| + 1,0 | 5 |

Abb. 7

Richtungsverteilung

(a) für 2-dimensionale Richtungsmessung    (b) für 3-dimensionale Richtungsmessung

$$X(\omega)$$

$$X(\omega, h)$$

Abb. 8a                                       Abb. 8b

LUT

für Symmetrieklasse
aus CeBr$_3$-Plastik Detektorpaaren          Winkel $\vartheta$ →
Nuklid: Co-60

$$A = \frac{E2 - E1}{E1 + E2}$$

| A | 0° | 5° | 10° | 15° | … | 180° |
|---|---|---|---|---|---|---|
| - 1,0 | 21 | 13 | 12 | 10 | | 11 |
| - 0,9 | 85 | 60 | 70 | 101 | | 35 |
| - 0,8 | 137 | 114 | 127 | 126 | | 63 |
| - 0,7 | 67 | 79 | 84 | 84 | | 112 |
| - 0,6 | 48 | 61 | 41 | 54 | | 40 |
| - 0,5 | 46 | 44 | 36 | 46 | | 19 |
| - 0,4 | 24 | 39 | 38 | 52 | | 22 |
| - 0,3 | 28 | 34 | 34 | 45 | | 17 |
| - 0,2 | 27 | 30 | 36 | 31 | | 16 |
| - 0,1 | 20 | 35 | 35 | 41 | | 5 |
| 0,0 | 32 | 49 | 42 | 62 | | 2 |
| + 0,1 | 31 | 46 | 39 | 81 | | 10 |
| + 0,2 | 47 | 58 | 80 | 98 | | 10 |
| + 0,3 | 75 | 85 | 111 | 145 | | 13 |
| + 0,4 | 108 | 98 | 154 | 178 | | 13 |
| + 0,5 | 181 | 204 | 196 | 210 | | 11 |
| + 0,6 | 251 | 265 | 273 | 240 | | 19 |
| + 0,7 | 322 | 324 | 291 | 256 | | 25 |
| + 0,8 | 314 | 268 | 221 | 197 | | 30 |
| + 0,9 | 283 | 132 | 176 | 104 | | 15 |
| + 1,0 | | | | | | |

Abb. 9

Abb. 10

Abb. 11

Messplan mit Winkelpositionen einer Referenzquelle zur Erstellung der LUT

$$\vartheta = \arccos\left(\cos(\omega - \varphi_i)\right) \quad \rightarrow$$

| $\vartheta$ | 0° | 5° | 10° | 15° | 20° | 25° | 30° | 35° | 40° | 45° | 50° | 55° | |
|---|---|---|---|---|---|---|---|---|---|---|---|---|---|
| ID | I | I | I | I | I | I | I | II | II | II | II | II | |
| $\varphi_i$ | 0° | 0° | 0° | 0° | 0° | 0° | 0° | 60° | 60° | 60° | 60° | 60° | |
| $\omega$ | 0° | 5° | 10° | 15° | 20° | 25° | 30° | 25° | 20° | 15° | 10° | 5° | |

| $\vartheta$ | 60° | 65° | 70° | 75° | 80° | 85° | 90° | 95° | 100° | 105° | 110° | 115° | |
|---|---|---|---|---|---|---|---|---|---|---|---|---|---|
| ID | VI | VI | VI | VI | VI | VI | VI | III | III | III | III | III | |
| $\varphi_i$ | 300° | 300° | 300° | 300° | 300° | 300° | 300° | 120° | 120° | 120° | 120° | 120° | |
| $\omega$ | 0° | 5° | 10° | 15° | 20° | 25° | 30° | 25° | 20° | 15° | 10° | 5° | |

| $\vartheta$ | 120° | 125° | 130° | 135° | 140° | 145° | 150° | 155° | 160° | 165° | 170° | 175° | 180° |
|---|---|---|---|---|---|---|---|---|---|---|---|---|---|
| ID | V | V | V | V | V | V | V | IV | IV | IV | IV | IV | IV |
| $\varphi_i$ | 240° | 240° | 240° | 240° | 240° | 240° | 240° | 180° | 180° | 180° | 180° | 180° | 180° |
| $\omega$ | 0° | 5° | 10° | 15° | 20° | 25° | 30° | 25° | 20° | 15° | 10° | 5° | 0° |

Abb. 12

Abb. 13

Abb. 14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120043467 A

- US 8461547 B2

**In der Beschreibung aufgeführte Nicht-Patentliteratur**

- A novel method to determine the directionality of radiation sources with two detectors based on coincidence measurements. **GUEORGUIEV A et al.** NUCLEAR SCIENCE SYMPOSIUM CONFERENCE RECORD (NSS/MIC). IEEE, 30 October 2010, 1525-1530